# EUROPEAN PATENT APPLICATION

(11) **EP 4 147 705 A1**
(43) Date of publication of application: **15.03.2023**
(21) Application number: 21196600.7
(22) Date of filing: 14.09.2021
(51) Int. Cl.: A61K 36/185, A23L 33/00, A61P 1/08, A61P 15/00, A61P 21/02, A61P 25/00, A61P 25/02, A61P 25/18, A61P 25/20, A61P 25/28, A61P 31/00, B01D 11/02

(54) **METHOD FOR OBTAINING BIOACTIVE COMPONENTS FROM A CANNABINOID-POOR AND/OR TERPENE-POOR WASTE STREAM COMPRISING CANNABIS FLOWER AND/OR CANNABIS LEAF MATERIAL**

(71) Applicant: De Wit, Marcel, 6300 Zug (CH)
(72) Inventor: De Wit, Marcel, 6300 Zug (CH)
(74) Representative: V.O.

(57) **Abstract**

The invention relates to a method for obtaining one or more biologically active components other than a cannabinoid and/or a terpene from a cannabinoid-poor and/or terpene-poor waste product comprising Cannabis flower and/or Cannabis leaf material, comprising contacting the cannabinoid-poor and/or terpene-poor waste product comprising Cannabis flower and/or Cannabis leaf material with a polar solvent, thereby obtaining an extract comprising one or more biologically active components and optionally isolating said one or more biologically active components from said extract.

## Description

### FIELD

The invention relates to a method for obtaining bioactive components from a cannabinoid-poor and/or terpene-poor waste stream comprising Cannabis flower and/or Cannabis leaf material.

### INTRODUCTION

The medicinal use of cannabis has a long history of several thousands of years and has been reported in the ancient Ayurvedic and Chinese Medicine (Pisanti and Bifulco, 2019. J. Cell Physiol. 234:8342-8351). Although cannabis was introduced in the modern western medicine in the 19^{th} century, the real breakthrough came with the discovery of the cannabinoids by the group of Mechoulam (Mechoulam and Gaoni, 1967. Fortschr. Chem. Org. Naturst. 25:175-213) and the discovery of the endocannabinoid systems with its receptors (Pertwee, 1993. Gen. Pharm. 24:811-824; Mackie, 2008. J. Neuroendocrinol. 20:10-14.). From that moment on research activities and clinical applications of the medicinal cannabis was mainly focussed on the major cannabinoid Δ⁹-tetrahydrocannabinol (THC) and cannabinol (CBD) later on. As a consequence, purified and synthetic THC were produced by the pharmaceutical industry for a broad scale of medicinal applications.

However, based on experiences from patients, it became more and more clear that cannabis extracts might be more beneficial as compared to purified or synthetic cannabinoids. For example, for Multiple Sclerosis it has been demonstrated that cannabis extracts containing a full spectrum of metabolites like cannabinoids, terpenoids and flavonoids were more beneficial in clinical applications as compared to the purified cannabinoids like THC/CBD or pharmaceutical products thereof (Maaya 2020, Inflamm. Res. 2020 69(6):549-558).

The first scientific reports on the putative entourage effect of cannabis in which other compounds like terpenoids and flavonoids were involved, appeared in the late nineties (Mechoulam, 1998, Eur J Pharmacol. 359:1-18; McPartland and Russo, 2001. J. Cannabis Therapeutics 1:103-132; Russo, 2011. Br. J. Pharmacol. 163: 1344-1364).

Since cannabinoids, terpenoids and flavonoids all belong to the class of secondary metabolites, the content of these compounds is strongly dependent on both the genotype and growing- and processing conditions of Cannabis plants. This explains why some patients experience having a strong preference for an extract obtained from a specific cultivar for use in the treatment of a certain medicinal indication. Although this information is still anecdotal and rather subjective without any clinical evidence, a recent study among 100.000 users and more than 1200 cannabis cultivars revealed a correlation between the metabolite content and consumers perception (de la Fuente et al, 2020; J. Cannabis Res. . 2020;2(1):2). However objective scientific data supporting a strong correlation between the cannabis genotype/chemotype and the medicinal effects is still very limited.

Up to now, the focus has been strongly directed to isolating aliphatic compounds, such as cannabinoids, such as THC and CBD, and/or terpenes from Cannabis, which are linked to a variety of medicinal effects. Typically these hydrophobic compounds are isolated from Cannabis by extraction using an non-polar solvent such as hexane.

The inventors found that from a waste stream of Cannabis flower and/or Cannabis leaf material, from which commercially and therapeutically relevant compounds cannabinoids and/or terpenes have been isolated, a polar extract can be obtained that exhibits biological activity.

It has been surprisingly realized that said biological activity was not induced by cannabinoids and/or terpenes, as polar extracts that were essentially free of cannabinoids and/or terpenes still exhibited significant biological activity.

Accordingly, the invention relates to a method for obtaining one or more biologically active components from a cannabinoid-poor and/or terpene-poor waste stream comprising Cannabis flower and/or Cannabis leaf material, comprising contacting a cannabinoid-poor and/or terpene-poor waste stream comprising Cannabis flower and/or Cannabis leaf material with a polar solvent, thereby obtaining an extract comprising one or more biologically active components and optionally isolating said one or more biologically active components from said extract.

The invention further relates to an extract or one or more biologically active components obtainable by a method according to the invention, and to a pharmaceutical composition comprising one or more biologically active components according to the invention.

### FIGURE LEGENDS

Figure 1: Principale component analysis of the ethanol extract obtained from flowertops of 13 different varieties based on NMR profiles.
Figure 2: Pharyngeal pumping effects of ethanol extracts obtained from flower tops of Cannabis cultivars MGC 1101 and MGC 1013, compared to control. Cultivar MGC 1013 showed a significant decrease of pharyngeal pumping activity whereas cultivar MGC 1101 showed an increase. A combination of both neutralized the effects.
Figure 3: Pharyngeal pumping effects of ethanol extracts obtained from flower tops of other Cannabis cultivars that did not show any effect on pharyngeal pumping activity, indicating that the effect of MGC 1101 and MGC 1013 are cultivar specific.
Figure 4: Pharyngeal pumping effects were observed for ethanol extracts obtained from flower tops of cultivar MGC 1013 in a dose-dependent manner. The non-polar extracts (NP) both heated and non-heated showed dose dependent decrease of the pharyngeal pumping activity but this was due to toxicity effects (figure 6-12).
Figure 5: Pharyngeal pumping effects were observed for ethanol extracts obtained from flower tops of cultivar MGC 1101 in a dose-dependent manner. The non-polar extracts (NP) both heated and non-heated showed dose dependent increase of the pharyngeal pumping activity but this was due to toxicity effects (figure 6-12)-
Figure 6: Longevity test of nematodes exposed to milli-Q water or DMSO.
Figure 7: Longevity test of nematodes exposed to different concentrations of the non-polar extract of MGC 1013 compared to control (solvent) DMSO. The results indicate that NP extracts of MGC 1013 are toxic to nematodes.
Figure 8: Longevity test of nematodes exposed to different concentrations of the ethanol extract of MGC 1013 compared to control (solvent) DMSO.
Figure 9: Longevity test of nematodes exposed to different concentrations of the non-polar heated extract of MGC 1013 compared to control (solvent) DMSO. The results indicate that NP heated extracts of MGC 1013 are toxic to nematodes.
Figure 10: Longevity test of nematodes exposed to different concentrations of the non-polar extract of MGC 1101 compared to control (solvent) DMSO. The results indicate that NP extracts of MGC 1101 are toxic to nematodes.
Figure 11: Longevity test of nematodes exposed to different concentrations of the non-polar heated extract of MGC 1101 compared to control (solvent) DMSO.
Figure 12: Longevity test of nematodes exposed to different concentrations of the ethanol extract of MGC 1101 compared to control (solvent) DMSO.
Figure 13 : Pharyngeal pumping effects were observed for ethanol extracts obtained from flower tops (#1 and #2) of cultivar MGC 1013 dissolved in DMSO, compared to control.
Figure 14: Pharyngeal pumping effects were observed for ethanol extracts obtained from flower tops (#1 and #2) of cultivar MGC 1101 dissolved in DMSO, compared to control.
Figure 15: Pharyngeal pumping is affected in nematodes of 12 days old by MGC 1101 and MGC 1013 in a dose responsive manner.
Figure 16: Touch response of old nematodes (12 days) grown in presence 5, 50 and 100 pg/mL of an ethanol extract obtained from cultivar MGC 1101 and MGC 1013 compared to control. A slower touch response was observed for nematodes grown in presence of an ethanol extract obtained from cultivar MGC 1101.
Figure 17: Motility test based on the number of oscillations per minute in young nematodes grown in presence of 100 pg/mL of an ethanolic extract for cultivars MGC 1003, MGC 1106, MGC 1007, MGC 1009, MGC 1101, MGC 1013, MGC 1027 and MGC 1046. The cultivars MGC 1003, MGC 1007, MGC 1009, MGC 1101, MGC 1027 and MGC 1046 showed reduced motility indicating a body-relaxing effect.
Figure 18: Response of nematodes to noxious smell of 1-octanol in presence of 5 and 100 pg/ml ethanol extract of cultivar MGC 1003, MGC 1106, MGC 1007, MGC 1009, MGC 1101, MGC 1013 and MGC 1027 and MGC 1046 compared to control. A slower response was observed for nematodes grown in presence of an extract obtained from cultivar MGC 1003, MGC 1007, MGC 1009, MGC 1013 and MGC 1027.
Figures 19-20: Response of nematodes to noxious smell of 1-octanol in presence of 100 pg/ml ethanol extract obtained from cultivar MGC 1122 and MGC 1074. A slower response was observed for both cultivars (Figure 19), whereas the general motility based on the number of oscillations remain the same (Figure 20).
Figure 21: Endurance assay: Swimming experiment where nematodes were exhausted during a swimming period (swim control) in the presence of 100 µg/mL ethanolic extract of cultivars MGC 1013 and MGC 1106. Exposure to both cultivars resulted in a higher endurance as compared to the control solvent (ethanol).
Figure 22: Survival rate of nematodes in response to heat shock in presence of an extract obtained from cultivars MGC 1101, MGC 1013 and MGC 1024 compared to control.
Figure 23: Survival rate of nematodes in response to heat shock in presence of an extract obtained from cultivars MGC 1104, MGC 1010 and MGC 1009, compared to control. A higher survival rate was observed for nematodes grown in presence of an ethanol extract obtained from cultivars MGC 1104 and MGC 1009.

### SUMMARY OF THE INVENTION

Provided herein is a method for obtaining one or more biologically active components other than a cannabinoid and/or a terpene from a cannabinoid-poor and/or terpene-poor waste product comprising Cannabis flower and/or Cannabis leaf material, comprising contacting the cannabinoid-poor and/or terpene-poor waste product comprising Cannabis flower and/or Cannabis leaf material with a polar solvent, thereby obtaining an extract comprising one or more biologically active components and optionally isolating said one or more biologically active components from said extract.

It was advantageously found that from cannabinoid-poor and/or terpene-poor waste material, one or more biologically active components other than a cannabinoid and/or a terpene could be extracted, that exhibit(s) valuable therapeutic or nutritional effects. Notably, said one or more biologically active components and corresponding activity appeared to be interrelated to the variety of Cannabis plant said Cannabis flower and/or leaf material is obtained from.

Accordingly, said Cannabis flower and/or leaf material comprises Cannabis flowers, Cannabis leaves or parts thereof are preferably obtained from the species *Cannabis sativa* L, more preferably from the plants MGC 1002, MGC 1003, MGC 1007, MGC 1008, MGC 1009, MGC 1010, MGC 1011, MGC 1013, MGC 1014, MGC 1015, MGC 1018, MGC 1020, MGC 1022, MGC 1023, MGC 1024, MGC 1026, MGC 1027, MGC 1030, MGC 1034, MGC 1036, MGC 1037, MGC 1038, MGC 1044, MGC 1046, MGC 1047, MGC 1050, MGC 1054, MGC 1064, MGC 1065, MGC 1066, MGC 1068, MGC 1069, MGC 1073 MGC 1074, MGC 1075, MGC 1085, MGC 1091, MGC 1094, MGC 1095, MGC 1097, MGC1101, MGC 1104, MGC 1106, MGC 1121, MGC 1122, MGC 1128, MGC 1138, MGC 1140, MGC 1143, MGC 1145, MGC 1150, MGC 1154, MGC 1155, MGC 1156, MGC 1157.

Further, said Cannabis flower and/or leaf material preferably comprises Cannabis flower buds or parts thereof, preferably dried Cannabis flower buds or parts thereof.

It was found that said extract comprising one or more biologically active components, or said isolated biologically active components exhibit valuable therapeutic or nutritional activity. Preferably, said one or more biologically active components comprises at least one component having a stimulating or a relaxing effect, more preferably a stimulating effect in enhancing appetite, energy, muscle function and/or brain activity, and/or a relaxing effect, more preferably relaxing muscle tension or brain activity, inhibition of nausea, inhibition of pain or inhibition of addiction.

Said polar solvent is preferably selected from the group consisting of water, aqueous buffer, methanol, ethanol, propanol, 2-propanol, butanol, 2-methyl-propanol, acetone, ethyl acetate, dimethyl sulfoxide, dimethylformamide, acetonitrile, 1,4-dioxane, tetrahydrofuran, or a combination thereof. Accordingly, said one or more biologically active components is preferably polar and/or water-soluble, more preferably wherein said one or more biologically active components has a water solubility of at least 1 mg per 100 mL.

Said cannabinoid-poor and/or terpene-poor waste product comprising Cannabis flower and/or leaf material is preferably obtained by contacting Cannabis flowers, leaves or parts thereof with an organic non-polar solvent, preferably an organic non-polar solvent is selected from the group consisting of n-pentane, n-hexane, n-heptane, n-octane, n-nonane, n-decane, n-dodecane, 2-methylpentane, 3-methylpentane, 2-methylhexane, 3-methylhexane, cyclopentane, cyclohexane, cycloheptane, cyclooctane, cyclodecane, cyclododecane, benzene, toluene, naphthalene, carbon tetrachloride, chloroform, dichloromethane, diethylether, supercritical carbon dioxide or a combination thereof, thereby obtaining a cannabinoid-rich and/or terpene-rich fluid and a cannabinoid-poor and/or terpene-poor waste product comprising Cannabis flower and/or leaf material, wherein said cannabinoid-poor and/or terpene-poor waste product preferably comprises less than 10 wt.% of cannabinoids and/or terpenes, based on the dry weight of said waste product, more preferably wherein said waste product is essentially free of cannabinoids and/or terpenes, as determinable with ¹H NMR spectroscopy.

Accordingly, said extract comprising one or more biologically active components further preferably comprises one or more cannabinoids and/or terpenes, wherein said cannabinoids and/or terpenes are preferably present in an amount of at most 10 wt.% of terpenes and/or at most 10 wt.% of cannabinoids, based on the total weight of said one or more biologically active components.

Preferably, said one or more biologically active component(s) is/are isolated by evaporation of said polar solvent from said extract.

The invention further relates to an extract comprising one or more biologically active components or one or more biologically active components obtainable by a method according to the invention.

In another aspect, the invention relates to a pharmaceutical or a nutritional composition comprising one or more biologically active components according to the invention and a pharmaceutically acceptable carrier.

The invention further relates to one or more biologically active components or a pharmaceutical composition according to the invention for use in a method of treatment by therapy.

Preferably, said one or more biologically active components or said pharmaceutical composition according to the invention is for use in the prevention or treatment of nausea, addiction, sleeping disorders, eating disorders, pain, sexual arousal and reproduction disorders, fatigue, muscle cramps, muscle spasms, tremors, tics, infections, inflammation, immune system defects, neurodegenerative disorders and behavioral disorders, preferably wherein said behavioral disorder is selected from the group consisting of major depression, bipolar disorder, anxiety disorder, attention deficit hyperactivity disorder (ADHD), attention deficit disorder (ADD), autism, schizophrenia, stress, burn-out, post-traumatic stress disorder and phobia.

The invention further relates to a use of the one or more biologically active components according to the invention as a relaxing agent, to increase energy, to increase focus, to enhance creativity, to enhance libido, or for increasing or decreasing body weight.

### DETAILED DESCRIPTION OF THE INVENTION

### Definitions

The term "or" as used herein is defined as "and/or" unless specified otherwise.

The term "a" or " an" as used herein is defined as "at least one" unless specified otherwise.

When referring to a noun in the singular, the plural is meant to be included, unless it follows from the context that it should refer to the singular only.

The term "substantial(ly)" or "essential(ly)" is generally used herein to indicate that it has the general character or function of that which is specified. When referring to a quantifiable feature, these terms are in particular used to indicate that it is for at least 75 %, more in particular at least 90 %, even more in particular at least 95 % of the indicated feature.

When referring to "essentially free", this generally means that a component is absent (not detectable), or - if present - in such low amount that it does not cause any significant effect. In particular, a composition is considered essentially free of a component, if the concentration is less than 0.1 mmol/l, more in particular less than 0.01 mmol/l in a liquid composition, and/or less than 0.1 wt.% of the dry weight of the composition, more in particular less than 0.01 wt.% of the dry weight, either in a liquid composition or in a dry product.

The term "poor" as in "cannabinoid-poor" or "terpene-poor" refers to a relatively low content of a substance in a medium, such as a waste-product, compared to the content of the same substance in a (natural) product said substance-poor medium is originates from. In the present context, a "cannabinoid-poor and/or terpene-poor waste product" in particular refers to a waste product that is obtained after subjecting Cannabis flowers, Cannabis leaves or parts thereof to a process wherein a cannabinoid and/or a terpene is removed from the flower and/or leaf material, more in particular by extraction. Said "cannabinoid-poor and/or terpene-poor waste product" typically comprises a lower amount of cannabinoids and/or terpenes (in wt.%, based on dry weight of the waste product), compared to the amount of said cannabinoids and/or terpene in the Cannabis flowers, leaves or parts thereof said waste product is obtained from (in wt.%, based on dry weight of the Cannabis flower, leaves or parts thereof).

Cannabis flowers, Cannabis leaves or parts thereof typically comprise between about 1 and about 25 wt.% of cannabinoids and/or between about 1 and about 2.5 wt.% of terpenes based on the total dry weight of said Cannabis flowers, Cannabis leaves or parts thereof, depending on the specific variety said flowers, leaves or part thereof are obtained from

With the term "cannabinoid" as used herein is meant a compound characterized by having a chemical structure as represented in Formula 1. 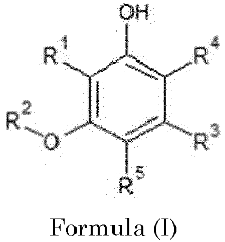
wherein, R¹ is an aliphatic moiety;
R² is a hydrogen or an aliphatic moiety;
R³ is an aliphatic moiety, preferably an aliphatic moiety selected from the group consisting of CH₃, CH₂CH₃, and CH₂(CH₂)ₙCH₃, wherein n is any integer number between 1 and 10, more preferably wherein R³ is CH₂(CH₂)ₙCH_{3;}
R⁴ is a hydrogen, a carboxylic acid or a carboxylate; and
R⁵ is a hydrogen, an hydroxyl group, a carboxylic acid, a carboxylate, OCOCH₃, OCOCH₂CH₃ or OCOCH₂CH₂CH₃.
wherein, the R¹ moiety and the adjacent hydroxyl group may be separate or may form an intramolecular four-, five-, six-, seven-, eight-, nine- or ten-membered ring or bicyclic structure, preferably a five- or six-membered ring and/or wherein the R¹ and R² moieties may be separate or may form an intramolecular four-, five-, six-, seven- or eight-, nine- or ten-membered ring or bicyclic structure.

Examples of cannabinoids include cannabidiol (CBD), also known under the IUPAC name 2-[(1*R*,6*R*)-6-isopropenyl-3-methylcyclohex-2-en-1-yl]-5-pentylbenzene-1,3-diol, Δ⁹-Tetrahydrocannabinol (THC) also known under the IUPAC name (6a*R*,10a*R*)-6,6,9-trimethyl-3-pentyl-6a,7,8,10a-tetrahydrobenzo[c]chromen-1-ol, and Δ⁹-Tetrahydrocannabinolic acid (THCa), also known under the IUPAC name (6a*R*,10*aR*)-1-hydroxy-6,6,9-trimethyl-3-pentyl-6a,7,8,10*a*-tetrahydrobenzo[c]chromene-2-carboxylic acid. Cannabinoids generally exert a psychoactive effect in a subject, when administered to a subject in an effective amount.

Cannabinoids are typically capable of binding a cannabinoid receptor. Known subtypes of the cannabinoid receptor in human include cannabinoid receptor 1 (CB1) and cannabinoid receptor 2 (CB2).

With the term "terpenes" as used herein is meant a compound build-up from 2-methylbutane residues, usually referred to as units of isoprene, which has the molecular formula C₅H₈ and is represented by formula II.

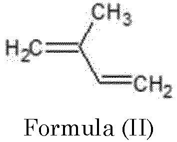

One can consider the isoprene unit as one of nature's common building blocks. The basic molecular formulae of terpenes are multiples of that formula: (C₅H₈)ₙ, wherein n is the number of linked isoprene units. This is called the isoprene rule, as a result of which terpenes are also denoted as isoprenoids. The isoprene units may be linked together "head to tail" to form linear chains or they may be arranged to form rings. In their biosynthesis, terpenes are formed from the universal 5 carbon precursors isopentenyl diphosphate (IPP) and its isomer, dimethylallyl diphosphate (DMAPP). Accordingly, a terpene carbon skeleton generally comprises a multiple of 5 carbon atoms. Most common are the 5-, 10-, 15-, 20-, 30- and 40-carbon terpenes, which are referred to as hemi-, mono-, sesqui-, di-, tri- and tetraterpenes, respectively. Besides "head-to-tail" connections, tri- and tetraterpenes also contain one "tail-to-tail" connection in their center.

The terpenes may comprise further functional groups, like alcohols and their glycosides, ethers, aldehydes, ketones, carboxylic acids and esters. These functionalized terpenes are herein referred to as "terpenoids" or "isoprenoids". Like terpenes, terpenoids generally have a carbon skeleton having a multiple of five carbon atoms. It should be noted that the total number of carbons in a terpenoid does not need to be a multiple of 5, *e.g.* the functional group may be an ester group comprising an alkyl radical having any number of carbon atoms.

Examples of terpenes include Myrcene, α-Pinene, β-Pinene, β-Caryophyllene, Linalool and Limonene.

Apart from the definitions given above, it is important to note that the terms "terpene", "terpenoid" and "isoprenoid" are frequently used interchangeably in open as well as patent literature.

The term "waste product", is generally known in the art, and refers to a secondary stream generated by a domestic or industrial process aimed to recover one or more valuable substances from a product, which secondary stream is typically subject to disposal. In the present context, a waste product comprising Cannabis flower and/or Cannabis leaf material, refers to a product generated upon extracting or otherwise treating Cannabis flowers, leaves or parts thereof to recover cannabinoids and/or terpenes therefrom, which product is substantially depleted in cannabinoids and/or terpenes. Said waste product comprising Cannabis flower and/or Cannabis leaf material typically comprises at least the solid remains obtained from Cannabis flowers, Cannabis leaves or parts thereof.

With the term "Cannabis flower material" as used herein, is meant any solid, semi-solid material or suspension obtained from Cannabis flowers or parts thereof.

With the term "Cannabis leaf material" as used herein, is meant any solid, semi-solid material or suspension obtained from Cannabis leaves or parts thereof.

With the term "cultivar" or "variety" as used herein, is meant a specific Cannabis plant or group of cannabis plants that is characterized by one or more specific properties, which can be maintained during breeding.

The term "polar" as used herein refers to a solvent that has a polarity index of 4 or more, as determinable by the method described in Snyder et al. (Snyder, 1974. J. Chrom. A 92:223-230). Examples of polar solvents include methanol (polarity index of 5.1), ethanol (polarity index 4.3) and water (polarity index 10.2). The term "non-polar" as used herein refers to a solvent that has a polarity index of less than 4 (Snyder, 1974. J. Chrom. A 92:223-230). Examples of non-polar solvents include heptane (polarity index of 0.1), hexane (polarity index of 0.1) and toluene (polarity index of 2.4).

With the term "biologically active component" as used herein, is meant a component that exerts a physiological effect in a subject, preferably a human, when provided at an effective amount. Such effect may be therapeutic or non-therapeutic.

In the context of the present invention, the term "biologically active component" typically comprises at least one component that is not present in an effective amount in another cultivar of Cannabis, of which an extract obtained therefrom does not exhibit said biological activity. The biological activity of a biological component is determinable using any suitable analytical technique known in the art, for example in a *C*. *elegans* assay or by empirical observation in humans, as described herein below.

A "therapeutic molecule" as used herein refers to any molecule that has a pharmaceutical benefit in a subject in need thereof, when provided at a therapeutically effective amount.

With the term "isolating" as used herein, is meant at least partly separating of a substance, particularly a biologically active component from its environment. "Isolating" encompasses separating said one or more biologically active components from said polar solvent to obtain said one or more biologically active components and optionally one or more further components. Said separation of said one or more biologically active components from said polar solvent may comprise evaporation of said polar solvent.

"Isolation" may further encompass removal of impurities and other substances until the one or more biologically active components are substantially pure.

"Substantially pure" is used herein to indicate that the one or more biologically active components have a purity of at least 95%, more preferably at least 96%, at least 97%, at least 98%, at least 99% or 100% pure, based on the total weight of the one or more biologically active components, as determined with standard analytical techniques known in the art on the date of filing.

With the term "pharmaceutically acceptable carrier" as used herein, is meant a carrier that has been approved by a health authority such as the European Medicines Agency or the Food and Drug Administration for *in vivo* use in mammals, specifically humans. Accordingly, a pharmaceutically acceptable carrier does typically not exert adverse effects to a mammalian body, e.g. is not substantially toxic.

### Methods

Cannabis flowers have long been known to be a rich source of compounds that are valued for their medicinal effect. These compounds have been identified as cannabinoids and may be extracted from Cannabis plants or parts thereof using a non-polar solvent such as hexane. It has now been found that the polar extract of Cannabis flowers and/or Cannabis leaves, which is low in cannabinoids and/or other non-polar constituents such as terpenes or steroids, preferably essentially free of cannabinoids and/or terpenes, comprises one or more biologically active components. Surprisingly, the biological activity of a polar extract obtained from Cannabis flower and/or Cannabis leaf material is not attributed to the presence of cannabinoids and/or terpenes, but to the presence of one or more other biologically active components present in said polar extract.

Accordingly, the invention relates to a method for obtaining one or more biologically active components other than a cannabinoid and/or a terpene from a cannabinoid-poor and/or terpene-poor waste product comprising Cannabis flower and/or Cannabis leaf material, comprising contacting a cannabinoid-poor and/or terpene-poor waste product comprising Cannabis flower and/or Cannabis leaf material with a polar solvent, thereby obtaining an extract comprising one or more biologically active components and optionally isolating said one or more biologically active components from said extract.

The method according to the invention advantageously allows obtaining an extract comprising one or more biologically active components, and/or isolation of said one or more biologically active components therefrom. Advantageously, said extract or said one or more biologically active components may be used as nutritional supplement or as therapeutic molecule in a subject in need thereof.

Said one or more biologically active components are obtained from a cannabinoid-poor and/or terpene-poor waste product comprising Cannabis flower and/or Cannabis leaf material.

Preferably, said cannabinoid-poor and/or terpene-poor waste product comprises at most 50 wt.% of cannabinoids, more preferably at most 40 wt.% of cannabinoids, even more preferably at most 30 wt.%, at most 20 wt.%, at most 15 wt.%, at most 10 wt.%, at most 9 wt.%, at most 8 wt.%, at most 7 wt.%, at most 6 wt.%, at most 5 wt.%, at most 4 wt.%, at most 3 wt.%, at most 2 wt.%, at most 1 wt.%, at most 0.5 wt.%, at most 0.3 wt.%, at most 0.2 wt.%, most preferably at most 0.1 wt.% of cannabinoids, based on the dry weight of said cannabinoid-poor and/or terpene-poor waste product, as determinable with ¹H NMR spectroscopy.

Preferably, said cannabinoid-poor and/or terpene-poor waste product comprises between about 0 and about 50 wt.% of cannabinoids, more preferably between about 0.1 and about 40 wt.%, between about 0.2 and about 30 wt.%, between about 0.3 and about 20 wt.%, between about 0.4 and about 15 wt.%, between about 0.5 and about 10 wt.%, between about 0.6 and about 9 wt.%, between about 0.7 and about 8 wt.%, between about 0.8 and about 7 wt.%, between about 0.9 wt.% and about 6 wt.%, between about 1 and about 5 wt.%, between about 1.5 and about 4 wt.%, between about 2 and about 3 wt.% of cannabinoids, based on the dry weight of said cannabinoid-poor and/or terpene-poor waste product.

Preferably, said cannabinoid-poor and/or terpene-poor waste product comprises at most 50 wt.% of terpenes, more preferably at most 40 wt.% of cannabinoids, even more preferably at most 30 wt.%, at most 20 wt.%, at most 15 wt.%, at most 10 wt.%, at most 9 wt.%, at most 8 wt.%, at most 7 wt.%, at most 6 wt.%, at most 5 wt.%, at most 4 wt.%, at most 3 wt.%, at most 2 wt.%, at most 1 wt.%, at most 0.5 wt.%, at most 0.3 wt.%, at most 0.2 wt.%, most preferably at most 0.1 wt.% of terpenes, based on the dry weight of said cannabinoid-poor and/or terpene-poor waste product, as determinable with ¹H NMR spectroscopy.

Preferably, said cannabinoid-poor and/or terpene-poor waste product comprises between about 0 and about 50 wt.% of terpenes, more preferably between about 0.1 and about 40 wt.%, between about 0.2 and about 30 wt.%, between about 0.3 and about 20 wt.%, between about 0.4 and about 15 wt.%, between about 0.5 and about 10 wt.%, between about 0.6 and about 9 wt.%, between about 0.7 and about 8 wt.%, between about 0.8 and about 7 wt.%, between about 0.9 wt.% and about 6 wt.%, between about 1 and about 5 wt.%, between about 1.5 and about 4 wt.%, between about 2 and about 3 wt.% of terpenes, based on the dry weight of said cannabinoid-poor and/or terpene-poor waste product.

Preferably, said cannabinoid-poor and/or terpene-poor waste product comprises at most 50 wt.% of a total of cannabinoids and terpenes, more preferably at most 40 wt.% of cannabinoids and terpenes, even more preferably at most 30 wt.%, at most 20 wt.%, at most 15 wt.%, at most 10 wt.%, at most 9 wt.%, at most 8 wt.%, at most 7 wt.%, at most 6 wt.%, at most 5 wt.%, at most 4 wt.%, at most 3 wt.%, at most 2 wt.%, at most 1 wt.%, at most 0.5 wt.%, at most 0.3 wt.%, at most 0.2 wt.%, most preferably at most 0.1 wt.% of a total of cannabinoids and terpenes, based on the dry weight of said cannabinoids and terpenes, as determinable with ¹H NMR spectroscopy.

Preferably, said cannabinoid-poor and/or terpene-poor waste product comprises between about 0 and about 50 wt.% of a total of cannabinoids and terpenes, more preferably between about 0.1 and about 40 wt.%, between about 0.2 and about 30 wt.%, between about 0.3 and about 20 wt.%, between about 0.4 and about 15 wt.%, between about 0.5 and about 10 wt.%, between about 0.6 and about 9 wt.%, between about 0.7 and about 8 wt.%, between about 0.8 and about 7 wt.%, between about 0.9 wt.% and about 6 wt.%, between about 1 and about 5 wt.%, between about 1.5 and about 4 wt.%, between about 2 and about 3 wt.% of a total of cannabinoids and terpenes, based on the total dry weight of said cannabinoid-poor and/or terpene-poor waste product.

Said cannabinoid-poor and/or terpene-poor waste product preferably comprises a low content of cannabidiol (CBD), cannabidiolic acid (CBDA), cannabigerol (CBG), cannabigerolic acid (CBGA), Δ-9-tetrahydrocanabinol (THC) and/or Δ-9-tetrahydrocanabinoic acid (THCA). Preferably, the total content of CBD, CBDA, CBG, CBGA, THC and THCA in said cannabinoid-poor and/or terpene-poor waste product is at most 50 wt.% of THC, CBD, CBDA, CBG, CBGA and THCA, more preferably at most 40 wt.% of THC, THCA, CBD, CBG, CBGA and CBD, even more preferably at most 30 wt.%, at most 20 wt.%, at most 15 wt.%, at most 10 wt.%, at most 9 wt.%, at most 8 wt.%, at most 7 wt.%, at most 6 wt.%, at most 5 wt.%, at most 4 wt.%, at most 3 wt.%, at most 2 wt.%, at most 1 wt.%, at most 0.5 wt.%, at most 0.3 wt.%, at most 0.2 wt.%, most preferably at most 0.1 wt.%, based on the dry weight of said cannabinoid-poor and/or terpene-poor waste product, as determinable with ¹H NMR spectroscopy.

In particular, the content of the total CBD, CBDA, CBG, CBGA, THC and THCA is comprises between about 0 and about 50 wt.% of THC, CBD, CBDA, CBG, CBGA and THCA, more preferably between about 0.1 and about 40 wt.%, between about 0.2 and about 30 wt.%, between about 0.3 and about 20 wt.%, between about 0.4 and about 15 wt.%, between about 0.5 and about 10 wt.%, between about 0.6 and about 9 wt.%, between about 0.7 and about 8 wt.%, between about 0.8 and about 7 wt.%, between about 0.9 wt.% and about 6 wt.%, between about 1 and about 5 wt.%, between about 1.5 and about 4 wt.%, between about 2 and about 3 wt.%, based on the total dry weight of said cannabinoid-poor and/or terpene-poor waste product.

Said cannabinoid-poor and/or terpene-poor waste product is preferably obtained by contacting Cannabis flowers, leaves or parts thereof with an organic non-polar solvent, thereby obtaining a cannabinoid-rich and/or terpene-rich fluid and a cannabinoid-poor and/or terpene-poor waste product comprising Cannabis flower and/or leaf material. Said cannabinoid-rich and/or terpene-rich fluid is typically recovered, and subsequently used for commercial or therapeutic purposes, e.g. as source of cannabinoid and/or terpenes.

Said organic non-polar solvent is typically a solvent having a polarity index of less than 4 as determinable by the method described in Snyder, 1974. J. Chrom. A 92:223-230. Preferably, said organic non-polar solvent has a low boiling point. This facilitates removal of residues of said non-polar solvent from said cannabinoid-poor and/or terpene-poor waste product comprising Cannabis flower and/or leaf material. Preferably, the boiling point of said non-polar solvent is at most 200 °C, more preferably at most 150 °C, at most 120 °C, at most 100 °C, at most 90 °C, at most 80 °C, in particular at most 70 °C. Typically, the boiling point of said non-polar solvent is between about 35 °C and about 150 °C, more preferably between 40 °C and 100 °C.

Preferably, said organic non-polar solvent is selected from the group consisting of n-pentane, n-hexane, n-heptane, n-octane, n-nonane, n-decane, n-dodecane, 2-methylpentane, 3-methylpentane, 2-methylhexane, 3-methylhexane, cyclopentane, cyclohexane, cycloheptane, cyclooctane, cyclodecane, cyclododecane, benzene, toluene, naphthalene, carbon tetrachloride, chloroform, dichloromethane, diethylether, petroleum ether, kerosene, supercritical carbon dioxide or a combination thereof.

Said contacting may be achieved using any suitable method known in the art, such as by incubating said Cannabis flowers, Cannabis leaves or parts thereof in said non-polar solvent for a sufficient amount of time, followed by filtration of said Cannabis flower and/or Cannabis leaf material over a suitable filter. Preferably, said contacting is achieved by extraction of Cannabis flowers, Cannabis leaves or parts thereof in an extraction cell (Bayona et al., 2018. Mar. drugs 16:393).

Preferably, said contacting, such as extraction takes place at elevated temperature, to increase the extraction of cannabinoids and/or terpenes into a non-polar solvent. Preferably, contacting, more preferably extraction takes place at a temperature of at least 20 °C, at least 25 °C, at least 30 °C, at least 40 °C, at least 45 °C. Such temperatures enhance extraction of cannabinoids and/or terpenes into the organic non-polar solvent.

Said contacting, preferably said extraction, typically takes place at a temperature of at most 100 °C, at most 90 °C, at most 80 °C, at most 70 °C, at most 60 °C, or at most 50 °C. Such temperatures ensure sufficient extraction of cannabinoids and/or terpenes into an organic non-polar solvent, whilst still being energetically favorable, thereby decreasing costs, reducing the risk of detoriation of the equipment and improving safety to the user.

In particular, said contacting, preferably said extraction, takes place at a temperature of between about 30 °C and about 60 °C, preferably between about 40 °C and about 50 °C, in particular about 45 °C. The skilled person will understand that the temperature depends on both the boiling point of the organic non-polar solvent and the conditions used during extraction. The temperature should preferably be selected such as to avoid substantial evaporation of said organic non-polar solvent during contacting.

Typically, contacting, such as extraction takes place at increased pressure, preferably at 5 bar or more, 10 bar or more, 20 bar, 30 bar or more, 40 bar or more, 50 bar or more, 60 bar or more, 70 bar or more, 80 bar or more, 90 bar or more, 100 bar or more, 150 bar or more, 200 bar or more.

Preferably, contacting, more preferably extraction takes place at between about 50 and about 150 bar, more preferably between about 80 and about 120 bar, in particular at about 100 bar.

Said contacting, such as extraction, takes place for an amount of time that is sufficient to extract a substantial amount of cannabinoids and/or terpenes into said non-polar solvent. Preferably, contacting takes place for at least 10 seconds, more preferably at least 20 seconds, at least 30 seconds, at least 40 seconds, at least 50 seconds, at least 60 seconds.

Typically, said contacting takes place for between about 10 seconds to about 300 seconds, preferably between about 30 seconds and about 240 seconds, between about 40 seconds and about 180 seconds, more preferably between about 60 seconds and about 120 seconds.

Said contacting may be repeated more than once, to ensure extraction of essentially all cannabinoids and/or terpenes from said Cannabis flowers, Cannabis leaves or parts thereof. Typically, said Cannabis flower, Cannabis leaves or parts thereof are contacted between one and five times, more preferably between two and four times to ensure sufficient extraction of said cannabinoids and/or terpenes from said Cannabis flowers, Cannabis leaves or parts thereof.

Said cannabinoid-poor and/or terpene-poor waste product comprises Cannabis flower and/or Cannabis leaf material.

Preferably Cannabis flower and/or leaf material comprises Cannabis flowers, Cannabis leaves or parts thereof obtained from the species *Cannabis sativa* L. Examples of *Cannabis sativa* L. include *C. sativa* subsp. *sativa, C. sativa* subsp. *indica, C. sativa* subsp. *ruderalis* and hybrids thereof.

The inventors realized that the biological activity of polar extracts obtained using a method according to the invention from Cannabis flower and/or Cannabis leaf material, was dependent on the Cannabis variety from which the cannabis flower and/or cannabis leaf material was obtained.

Therefore, without wishing to be bound by any theory, it is believed that specific Cannabis varieties comprise one or more unique biologically active components in their flowers and/or leaves, which are not present in an effective amount in in other Cannabis varieties of which a polar extract does not exhibit the same biological activity.

Good results have been obtained with Cannabis flower and/or leaf material or parts thereof obtained from Cannabis varieties MGC 1002, MGC 1003, MGC 1007, MGC 1008, MGC 1009, MGC 1010, MGC 1011, MGC 1013, MGC 1014, MGC 1015, MGC 1018, MGC 1020, MGC 1022, MGC 1023, MGC 1024, MGC 1026, MGC 1027, MGC 1030, MGC 1034, MGC 1036, MGC 1037, MGC 1038, MGC 1044, MGC 1046, MGC 1047, MGC 1050, MGC 1054, MGC 1064, MGC 1065, MGC 1066, MGC 1068, MGC 1069, MGC 1073 MGC 1074, MGC 1075, MGC 1085, MGC 1091, MGC 1094, MGC 1095, MGC 1097, MGC1101, MGC 1104, MGC 1106, MGC 1121, MGC 1122, MGC 1128, MGC 1138, MGC 1140, MGC 1143, MGC 1145, MGC 1150, MGC 1154, MGC 1155, MGC 1156, MGC 1157.

The invention further relates to a method as described herein, wherein said Cannabis flower and/or leaf material comprises Cannabis flowers, Cannabis leaves or parts thereof obtained from the species *Cannabis sativa* L, preferably a Cannabis variety chosen from the group selected from MGC 1002, MGC 1003, MGC 1007, MGC 1008, MGC 1009, MGC 1010, MGC 1011, MGC 1013, MGC 1014, MGC 1015, MGC 1018, MGC 1020, MGC 1022, MGC 1023, MGC 1024, MGC 1026, MGC 1027, MGC 1030, MGC 1034, MGC 1036, MGC 1037, MGC 1038, MGC 1044, MGC 1046, MGC 1047, MGC 1050, MGC 1054, MGC 1064, MGC 1065, MGC 1066, MGC 1068, MGC 1069, MGC 1073 MGC 1074, MGC 1075, MGC 1085, MGC 1091, MGC 1094, MGC 1095, MGC 1097, MGC1101, MGC 1104, MGC 1106, MGC 1121, MGC 1122, MGC 1128, MGC 1138, MGC 1140, MGC 1143, MGC 1145, MGC 1150, MGC 1154, MGC 1155, MGC 1156, MGC 1157. more preferably a Cannabis variety chosen from the group selected from MGC 1101, MGC 1003, MGC 1104, MGC 1106, MGC 1007, MGC 1009, MGC 1010, MGC 1013, MGC 1023, MGC 1024, MGC 1122, MGC 1074 and MGC 1046

Preferably, said Cannabis flower and/or leaf material comprises Cannabis flower buds or parts thereof, more preferably dried Cannabis flower buds or parts thereof. A dried Cannabis flower bud or parts thereof typically has a water content in the range of between about 0 and about 12 wt.%, preferably between about 0.1 and about 10 wt.%, more preferably between about 0.2 wt.% and about 8 wt.%, between about 0.5 wt.% and about 6 wt.%, between about 1 and about 4 wt.%, between about 2 and about 3 wt.% of water, based on the total weight of the dried Cannabis flower buds.

Said Cannabis flower and/or leaf material may be dried using any suitable method known in the art, for example by leaving said material to dry at ambient conditions, such as at a temperature of around 20 °C and at atmospheric pressure, for a sufficient amount of time. Alternatively, said Cannabis flower and/or leaf material may be dried at elevated temperature, such as at a temperature of more than 30 °C, preferably more than 40 °C, more than 50 °C, more than 60 °C. Alternatively or additionally, said Cannabis flower and/or leaf material may be dried at reduced pressure, such as 0.90 bar or lower, 0.80 bar or lower, 0.70 bar or lower, 0.60 bar or lower, 0.50 bar or lower, 0.40 bar or lower, 0.30 bar or lower, 0.20 bar or lower, 0.10 bar or lower, such as 0.05 bar or lower. Such conditions speed up the drying process.

Said Cannabis leaf material may comprise fan leaves and/or sugar leaves, but preferably comprises at least the sugar leaves. As is known to the skilled person, the sugar leaves are small leaves located in close proximity to the flowers of the Cannabis plant, whereas the fan leaves are typically much larger in size, have a multi-fingered shape, and attached directly to the stem of the Cannabis plant.

Preferably said Cannabis flower and/or Cannabis leaf material is essentially free of cannabis stem material, and/or Cannabis seed material.

Said Cannabis flower and/or Cannabis leaf material may be obtained from male and/or female Cannabis plants, preferably a male and/or female *Cannabis sativa* L plants, but is preferably obtained from a female Cannabis plant, preferably a female *Cannabis sativa* L, more preferably a female *Cannabis sativa* L subs *sativa* plant.

Said cannabinoid-poor and/or terpene-poor waste product comprising cannabis flower and/or Cannabis leaf material is contacted with a polar solvent, thereby obtaining an extract comprising one or more biologically active components.

Said polar solvent is a solvent having a polarity index of 4 or more. Preferably, said polar solvent has a low boiling point, in particular, in case isolation of one or more biologically active components from an extract is desired. Preferably, the boiling point of said polar solvent is at most 200 °C, more preferably at most 150 °C, at most 120 °C, at most 100 °C, at most 90 °C, at most 80 °C, in particular at most 70 °C. Typically, the boiling point of said polar solvent is between about 35 °C and about 150 °C, more preferably between 40 °C and 100 °C.

Preferably, said polar solvent is selected from the group consisting of water, aqueous buffer, methanol, ethanol, propanol, 2-propanol, butanol, 2-methyl-propanol, acetone, ethyl acetate, dimethyl sulfoxide, dimethylformamide, acetonitrile, 1,4-dioxane, tetrahydrofuran, or a combination thereof.

Typically, if an aqueous buffer is used as a polar solvent, the pH varies from between 1 and 12, preferably between 2 and 10, more preferably between 4 and 8, in particular between 5 and 7.

Said one or more biologically active components are optionally isolated from said extract. Isolation may be achieved using any suitable technique known in the art, for example by evaporation, co-evaporation or distillation of said extract, thereby removing said polar solvent from said extract. Preferably, said isolation is achieved under an inert atmosphere, such as under nitrogen or argon atmosphere, to prevent potential degradation of said one or more biologically active components.

Optionally, said isolation comprises one or more purification steps to remove impurities and/or, in case more than one biologically active components are present, to separate one or more biologically active components from one or more other biologically active components.

Said purification may be achieved using any suitable method known in the art, and is highly dependent on the composition of said extract, as the skilled person will understand. Typically said one or more biological components may be purified using (flash) column chromatography, affinity chromatography or size-exclusion chromatography.

Preferably, said extract comprising one or more biologically active components, or said one or more biologically active components isolated therefrom comprises at most 50 wt.% of cannabinoids, more preferably at most 40 wt.% of cannabinoids, even more preferably at most 30 wt.%, at most 20 wt.%, at most 15 wt.%, at most 10 wt.%, at most 9 wt.%, at most 8 wt.%, at most 7 wt.%, at most 6 wt.%, at most 5 wt.%, at most 4 wt.%, at most 3 wt.%, at most 2 wt.%, at most 1 wt.%, at most 0.5 wt.%, at most 0.3 wt.%, at most 0.2 wt.%, most preferably at most 0.1 wt.% of cannabinoids, based on the weight of said one or more biologically active components, as determinable with ¹H NMR spectroscopy.

Preferably, said extract comprising one or more biologically active components, or said one or more biologically active components isolated therefrom comprises between about 0 and about 50 wt.% of cannabinoids, more preferably between about 0.1 and about 40 wt.%, between about 0.2 and about 30 wt.%, between about 0.3 and about 20 wt.%, between about 0.4 and about 15 wt.%, between about 0.5 and about 10 wt.%, between about 0.6 and about 9 wt.%, between about 0.7 and about 8 wt.%, between about 0.8 and about 7 wt.%, between about 0.9 wt.% and about 6 wt.%, between about 1 and about 5 wt.%, between about 1.5 and about 4 wt.%, between about 2 and about 3 wt.% of cannabinoids, based on the weight of said one or more biologically active components.

Preferably, said extract comprising one or more biologically active components, or said one or more biologically active components isolated therefrom comprises at most 50 wt.% of terpenes, more preferably at most 40 wt.% of cannabinoids, even more preferably at most 30 wt.%, at most 20 wt.%, at most 15 wt.%, at most 10 wt.%, at most 9 wt.%, at most 8 wt.%, at most 7 wt.%, at most 6 wt.%, at most 5 wt.%, at most 4 wt.%, at most 3 wt.%, at most 2 wt.%, at most 1 wt.%, at most 0.5 wt.%, at most 0.3 wt.%, at most 0.2 wt.%, most preferably at most 0.1 wt.% of terpenes, based on the weight of said one or more biologically active components, as determinable with ¹H NMR spectroscopy.

Preferably, said extract comprising one or more biologically active components, or said one or more biologically active components isolated therefrom comprises between about 0 and about 50 wt.% of terpenes, more preferably between about 0.1 and about 40 wt.%, between about 0.2 and about 30 wt.%, between about 0.3 and about 20 wt.%, between about 0.4 and about 15 wt.%, between about 0.5 and about 10 wt.%, between about 0.6 and about 9 wt.%, between about 0.7 and about 8 wt.%, between about 0.8 and about 7 wt.%, between about 0.9 wt.% and about 6 wt.%, between about 1 and about 5 wt.%, between about 1.5 and about 4 wt.%, between about 2 and about 3 wt.% of terpenes, based on the total weight of said one or more biologically active components.

Preferably, said extract comprising one or more biologically active components, or said one or more biologically active components isolated therefrom comprises at most 50 wt.% of a total of cannabinoids and terpenes, more preferably at most 40 wt.% of cannabinoids and terpenes, even more preferably at most 30 wt.%, at most 20 wt.%, at most 15 wt.%, at most 10 wt.%, at most 9 wt.%, at most 8 wt.%, at most 7 wt.%, at most 6 wt.%, at most 5 wt.%, at most 4 wt.%, at most 3 wt.%, at most 2 wt.%, at most 1 wt.%, at most 0.5 wt.%, at most 0.3 wt.%, at most 0.2 wt.%, most preferably at most 0.1 wt.% of a total of cannabinoids and terpenes, based on the weight of said one or more biologically active components, as determinable with ¹H NMR spectroscopy.

Preferably, said extract comprising one or more biologically active components, or said one or more biologically active components isolated therefrom comprises between about 0 and about 50 wt.% of cannabinoids and terpenes, more preferably between about 0.1 and about 40 wt.%, between about 0.2 and about 30 wt.%, between about 0.3 and about 20 wt.%, between about 0.4 and about 15 wt.%, between about 0.5 and about 10 wt.%, between about 0.6 and about 9 wt.%, between about 0.7 and about 8 wt.%, between about 0.8 and about 7 wt.%, between about 0.9 wt.% and about 6 wt.%, between about 1 and about 5 wt.%, between about 1.5 and about 4 wt.%, between about 2 and about 3 wt.% of cannabinoids and terpenes, based on the total weight of said one or more biologically active components isolated therefrom.

Said extract comprising one or more biologically active components, or said one or more biologically active components isolated therefrom preferably comprises a low content of cannabidiol (CBD), cannabidiolic acid (CBDA), cannabigerol (CBG), cannabigerolic acid (CBGA), Δ-9-tetrahydrocanabinol (THC) and Δ-9-tetrahydrocanabinoic acid (THCA). Preferably, the total content of CBD, CBDA, CBG, CBGA, THC and THCA in said extract comprising one or more biologically active components, or said one or more biologically active components isolated therefrom is at most 50 wt.% of a total of THC, THCA, CBDA, CBG, CBGA and CBD, more preferably at most 40 wt.%, even more preferably at most 30 wt.%, at most 20 wt.%, at most 15 wt.%, at most 10 wt.%, at most 9 wt.%, at most 8 wt.%, at most 7 wt.%, at most 6 wt.%, at most 5 wt.%, at most 4 wt.%, at most 3 wt.%, at most 2 wt.%, at most 1 wt.%, at most 0.5 wt.%, at most 0.3 wt.%, at most 0.2 wt.%, most preferably at most 0.1 wt.% of THC, THCA, CBDA, CBG, CBGA and CBD, based on said one or more biologically active components isolated therefrom, as determinable with ¹H NMR spectroscopy.

In particular, the content of the total CBD, CBDA, CBG, CBGA, THC and THCA comprises between about 0 and about 50 wt.%, more preferably between about 0.1 and about 40 wt.%, between about 0.2 and about 30 wt.%, between about 0.3 and about 20 wt.%, between about 0.4 and about 15 wt.%, between about 0.5 and about 10 wt.%, between about 0.6 and about 9 wt.%, between about 0.7 and about 8 wt.%, between about 0.8 and about 7 wt.%, between about 0.9 wt.% and about 6 wt.%, between about 1 and about 5 wt.%, between about 1.5 and about 4 wt.%, between about 2 and about 3 wt.%, based on the weight of said one or more biologically active components isolated therefrom.

The contents of cannabinoids and/or terpenes in the extract as defined herein above, are under the proviso that the content of said cannabinoids and/or terpenes in the extract or in said isolated one or more biologically active components is typically equal to or less than the content of said cannabinoids and/or terpenes in the waste product comprising Cannabis flower and/or Cannabis leaf material from which the component(s) have been obtained.

### Extract, one or more biologically active components and pharmaceutical compositions

The invention further relates to an extract, or one or more biologically active components obtainable by a method according to the invention.

Preferably, said one or more biologically active components is not a cannabinoid and/or a terpene.

The content of cannabinoids and/or terpenes in an extract according to the invention is preferably as defined herein above. Most preferably, said extract comprising one or more biologically active components is essentially free of terpenes and/or cannabinoids.

Said extract or said one or more biologically active components may comprise one biologically active component, or may comprise a mixture of multiple biologically active components. Accordingly, said extract or said one or more biologically active components may comprise one, two, three, four five, six, seven, eight, nine, ten, eleven, twelve, thirteen, fourteen, fifteen, sixteen, seventeen, eighteen, nineteen or twenty biologically active components.

Said extract or said one or more biologically active components preferably exhibits one or more biological activities selected from the group consisting of reducing or stimulating appetite, relaxing or stimulating the body, preferably muscles, relaxing or stimulating brain activity, enhancing energy, reducing pain, reducing nausea and/or reducing addition.

It was surprisingly found that the biological activity of said extract or said one or more biologically active components, is highly dependent on the specific variety of Cannabis plant from which said flowers and/or leaf material has been obtained. Thus, without wishing to be bound by any theory, it is believed that in different varieties of Cannabis plants, specific biologically active components are present that are deciding for the biological activity of an extract obtained from these varieties. Said components are thus not present in an effective amount in an extract obtained from a variety which does not exhibit said biological activity.

Accordingly, an extract or one or more biologically active components according to the invention, preferably comprises at least one variety-specific biologically active component. Such a component is directly linked to the biological activity observed for the extract, and is not present in an effective amount in an extract obtainable from Cannabis flowers and/or Cannabis leaves originating from a Cannabis variety that does not exhibit said biological activity. The chemical structure of said at least one variety-specific biologically active component may be determined using a metabolomics approach followed by ¹H NMR spectroscopy, gas chromatography-mass spectrometry (GC/MS), liquid chromatography-mass spectrometry (LC/MS), or combinations thereof, of fractions of an extract obtainable by a method according to the invention.

Without wishing to be bound by any theory, it is believed that said at least one variety-specific biologically active component comprises one or more polar components present in the flowers and/or leaves of Cannabis plants. For example, it may be a component belonging to the class of flavonoids and/or saccharides. Such components are likely retained in Cannabis flower and/or leaf material during extraction with a non-polar solvent, whereas they may be dissolved into a polar solvent upon contacting.

Accordingly, said extract or said one or more biologically active components according to the invention, preferably comprises at least one polar compound. Preferably, said at least one polar compound has good solubility in a polar solvent having a polarity index of 4 or more and/or poor solubility in a solvent having a polarity index of less than 4.

Preferably, the solubility in a polar solvent having a polarity index of 4 or more is at least 1 mg per 100 mL polar solvent, more preferably at least 2 mg per 100 mL polar solvent, at least 3 mg per 100 mL, at least 4 mg per 100 mL, at least 5 mg per 100 mL, at least 6 mg per 100 mL, at least 7 mg per 100 mL, at least 8 mg per 100 mL, at least 9 mg per 100 mL, at least 10 mg per 100 mL, at least 20 mg per 100 mL, at least 50 mg per 100 mL, at least 70 mg per 100 mL, most preferably at least 100 mg per 100 mL.

Preferably, solubility in a non-polar solvent having a polarity index of less than 4 is at most 100 mg per 100 mL non-polar solvent, preferably at most 50 mg per 100 mL, more preferably at most 10 mg per 100 mL, even more preferably at most 1 mg per 100 mL, 0.1 mg per 100 mL, in particular at most 0.01 mg per 100 mL of non-polar solvent.

Preferably, the content of polar components in an extract or one or more biologically active components according to the invention is at least 5 wt.%, based on the weight of said one or more biologically active components, more preferably at least 10 wt.%, at least 15 wt.%, at least 20 wt.%, at least 25 wt.%, at least 30 wt.%, at least 35 wt.%, at least 40 wt.%, at least 45 wt.%, at least 50 wt.%, at least 55 wt.%, at least 60 wt.%, at least 65 wt.%, at least 70 wt.%, at least 75 wt.%, at least 80 wt.%, at least 85 wt.%, at least 90 wt.%, at least 95 wt.%, most preferably 100 wt.% of polar compounds.

In an embodiment, said extract or said one or more biologically active components according to the invention comprises at least one flavonoid. Preferably, said extract or said one or more biologically active components according to the invention comprises at least 30 wt.% of flavonoids based on the total weight of the one or more biologically active components, more preferably at least 40 wt.%, at least 50 t.% at least 60 wt.%, at least 70 wt.%, at least 80 wt.%, at least 90 wt.%, or 100 wt.% of flavonoids, based on the total weight of the one or more biologically active components.

Preferably, said extract or said one or more biologically active components according to the invention comprises between 30 and 70 wt.%, such as between 40 and 60 wt.% of flavonoids.

In an embodiment, said extract or said one or more biologically active components according to the invention comprises at least a saccharide. Preferably, said extract or said one or more biologically active components according to the invention comprises at least 30 wt.% of saccharides based on the total weight of the one or more biologically active components, more preferably at least 40 wt.%, at least 50 t.% at least 60 wt.%, at least 70 wt.%, at least 80 wt.%, at least 90 wt.%, or 100 wt.% of saccharides, based on the total weight of the one or more biologically active components.

Typically, said extract or said one or more biologically active components according to the invention comprises between 30 and 70 wt.% saccharides, such as between 40 and 60 wt.% of saccharides.

In a specific embodiment, an extract or one or more biologically active components according to the invention, comprises between 0 and 10 wt.% of cannabinoids, preferably between 0 and 5 wt.% of cannabinoids, between 0 and 10 wt.% of terpenes, preferably between 0 and 5 wt.% of terpenes, between 20 and 70 wt.% of flavonoids, preferably between 30 and 50 wt.% of flavonoids and between 20 and 70 wt.% of saccharides, preferably between 30 and 50 wt.% of saccharides.

Said extract or said one or more biologically active components according to the invention is preferably water-soluble. Preferably, said extract or said one or more biologically active components according to the invention have a water-solubility of at least 1 mg per 100 mL water, more preferably at least 2 mg per 100 mL water, at least 3 mg per 100 mL, at least 4 mg per 100 mL, at least 5 mg per 100 mL, at least 6 mg per 100 mL, at least 7 mg per 100 mL, at least 8 mg per 100 mL, at least 9 mg per 100 mL, at least 10 mg per 100 mL, at least 20 mg per 100 mL, at least 50 mg per 100 mL, at least 70 mg per 100 mL, most preferably at least 100 mg per 100 mL.

Said extract or said one or more biologically active components may further comprise one or more polar compounds typically present in cannabinoid-depleted and/or terpene-depleted waste products.

Examples of such polar compounds include orientin, vitexin, isovitexin, apigenin, luteolin, quercetin, cannflavin A, cannflavin B, β-sitosterol, kaempferol, glycosides thereof, and combinations thereof.

Although such compounds may be present in an extract obtainable by a method according to the invention, it is believed that such compounds do not exhibit the typical biological activity as described herein at the concentration at which it is present. It is believed that such polar compounds are widely abundant in a wide range of plants including Cannabis plants, and therefore their presence does not explain the dependency of biological activity among Cannabis varieties.

The biological activity exhibited by an extract or one or more biological components obtainable by a method according to the invention, may be determined using any suitable analytical technique known in the art, for example using a *Caenorhabditis elegans* assay, or by evaluating the biological activity *in vivo* in humans, as described herein below.

*C. elegans* was found to constitute a promising model system for the assessment of biological activity of extracts or one or more biological components according to the invention. The nematode *Caenorhabditis elegans* (*C. elegans*) is a transparent organism that has been successfully used as a key model system in human disease genes discovery (Apfeld and Alper, 2018. Methods Mol Biol. 1706: 53-75). The organism contains a digestive-, nervous- and reproductive system, musculature and exhibits complex behaviours.

Although the use of *C*. *elegans* as a model system for medicinal cannabis studies is very limited so far, this might be due to the fact that homologues of the cannabinoid receptors CB1 and CB2 are missing in *C. elegans.*

However, it was recently shown that the endocannabinoids 2-arachidonoylglycerol (2-AG) and anandamide (AEA) exert multiple behavioural effects in *C*. *elegans* via the cannabinoid-like NeuroPeptide Receptor family member NPR-19 (Oakes et al., 2019. J. Neurosci. 39:4142-4152).

In addition the same research group showed that another endocannabinoid signalling pathway was present in *C. elegans* which was independent on the cannabinoid-like receptor pathway but acts via transient receptor potential vanilloid 1 (TRPV1) and transient receptor potential (TRP)-like channels (Oakes et al., 2019. J. Neurosci. 39:4142-4152). In human, there is also evidence that (endo)cannabinoid signalling does not merely occur through CB1 and CB2 receptors, but that other targets are involved such as the vanilloid-type TRP channels (Di Marzo and Piscitelli, 2015. Neurotherapeutics 12:692-698). These findings enable the use of *C*. *elegans* as an objective model system to test correlations between the cannabis varieties on the one hand and the medicinal and/or behavioural effect on the other hand.

The invention further relates to a pharmaceutical or nutritional composition comprising one or more biologically active components according to the invention and a pharmaceutically acceptable carrier.

Examples of suitable pharmaceutically acceptable carriers include liquid carriers such as water and aqueous buffer solutions, such as physiological saline, phosphate-buffered saline (PBS) or phosphate buffer, solid carriers such as lactose, calcium chloride, pectin and dextrin or a gaseous carrier. Examples of gaseous carriers include hydrocarbons such as butane, isobutane or propane, and fluorocarbons such as 1,1,1,2-tetrafluorethane.

Said pharmaceutical or nutritional composition may optionally comprise one or more pharmaceutically acceptable excipients, such as salts, wetting agents, flavoring agents, texturing agents, and stabilizers.

Said pharmaceutical or nutritional composition may be formulated in any suitable way, such as a solid, a semi-solid, a liquid, a powder, a tablet, a capsule, a solution, a suspension, an emulsion, a gel, a cream, a spray, an aerosol, a lotion, or a transdermal plaster. Preferably, the pharmaceutical composition is formulated as a powder, a capsule, a transdermal plaster or an aerosol.

### Medical use and methods of treatment

The invention further relates to one or more biologically active components or a pharmaceutical composition according to the invention for use in a method of treatment by therapy.

Further, the invention preferably relates to one or more biologically active components or a pharmaceutical composition according to the invention for use in the prevention or treatment of nausea, addiction, sleeping disorders, eating disorders, pain, sexual arousal and reproduction disorders, fatigue, muscle cramps, muscle spasms, tremors, tics, infections, inflammation, immune system defects, neurodegenerative disorders and behavioral disorders.

As substantiated herein above, it has been found that an extract or one or more biologically active components according to the invention, are strongly correlated to the Cannabis variety from which the extract has been obtained.

Said extract or one or more biologically active components according to the invention exhibit biological activity which is believed to be linked to one or more cultivar-specific biologically active components.

In particular, it was found that an extract or one or more biologically active components obtained from Cannabis flowers, Cannabis leaves or parts thereof of cultivar MGC 1101, according to the invention, surprisingly stimulates appetite in a subject to which said extract or said one or more biologically active components have been administered. This effect was confirmed in a *C*. *elegans* assay, wherein pharyngeal pumps of nematodes were significantly increased with approximately 20 to 50 pumps per minute, depending on the concentration of extract the nematodes were exposed to (see Example 2). This effect was further confirmed in over 60 humans, which experienced an increased feeling of hunger upon smoking of Cannabis flowers of cultivar MGC 1101 comprising said one or more biologically active components (Example 7).

Further, it was found, that an extract or one or more biologically active components obtained from Cannabis flowers, Cannabis leaves or parts thereof of cultivar MGC 1013, according to the invention, surprisingly inhibits appetite in a subject to which said extract or said one or more biologically active components have been administered. This was observed in a *C*. *elegans* assay, wherein pharyngeal pumps of nematodes were significantly decreased with approximately 20 to 60 pumps per minute, depending on the concentration of extract the nematodes were exposed to (see Example 2). This effect was further confirmed in over 30 humans, which experienced decreased feeling of hunger, and/or a premature feeling of satiety upon smoking of Cannabis flowers of cultivar MGC 1013 comprising said one or more biologically active components (Example 7).

Accordingly, in a particularly preferred embodiment, the invention relates to an extract or one or more biologically active components obtainable by a method according to the invention, wherein said Cannabis flower material and/or Cannabis leaf material was obtained from Cannabis cultivar MGC 1101 or MGC 1013 for use in a method of increasing or decreasing body weight in a subject in need thereof.

Said subject may be any subject in need of therapeutically increasing or maintaining body weight, but is preferably a mammal, more preferably a human.

Typically, a subject, preferably a human, in need of decreasing body weight according to the invention, has a BMI that is considered unhealthy due to a high risk of developing one or more diseases. Typically, a BMI of more than 30, preferably more than 35, even more preferably 40, is considered unhealthy overweight or obese. Such subjects are at risk of developing a wide range of diseases such as cardiovascular disease, diabetes type 2, hypertension, stroke, high cholesterol, gallbladder disease and cancer.

Further, a BMI of 18.5 or lower, preferably 18 or lower, more preferably 16 or lower is considered as unhealthy underweight. Subjects, preferably humans, having a BMI of 18.5 or lower are at risk of developing various diseases including osteoporosis, anemia, skin, hair or teeth problems, impaired immune system, exhaustion, fatigue, anemia or impaired growth.

Therefore, a subject, preferably a human, in need of increasing body weight according to the invention, is preferably a human having a BMI of 18.5 or lower, preferably 17 or lower, more preferably 16 or lower, or a human having a BMI of 30 or more, preferably 35 or more, in particular 40 or more.

Increasing or decreasing body weight in a subject in need thereof, may also be advantageous in the prevention or treatment of eating disorders. Accordingly, the invention further relates to an extract or one or more biologically active components obtainable by a method according to the invention, wherein said Cannabis flower material and/or Cannabis leaf material was obtained from Cannabis cultivar MGC 1101 or MGC 1013 for use in the prevention or treatment of eating disorders.

Examples of eating disorders include anorexia nervosa, bulimia nervosa, purging disorder, avoidance/restrictive food intake disorder (ARFID), other specified feeding or eating disorders, binge eating disorder, compulsive overeating, Prader-Willi syndrome, night eating syndrome and gourmand syndrome.

In another aspect, the subject in need of therapeutically increasing or maintaining body weight is a subject, preferably a human, that experiences a reduced feeling of hunger or appetite, preferably as a result of nausea or lack of appetite. Examples thereof include subjects undergoing treatment wherein possible side-effects include nausea and/or lack of appetite.

Preferably, a subject in need of therapeutically increasing or maintaining body weight according to the invention, is a subject, preferably a human, suffering from cancer, a human immunodeficiency virus (HIV)-positive subject, or a subject suffering from acquired immunodeficiency syndrome (AIDS).

Preferably, a subject in need of therapeutically increasing or maintaining body weight according to the invention is a subject, preferably a human, undergoing chemotherapy, radiotherapy or a subject receiving treatment for HIV/AIDS.

It was further found that an extract or one or more biologically active components obtainable by a method according to the invention, wherein said Cannabis flower material and/or Cannabis leaf material was obtained from Cannabis cultivar MGC 1101 and MGC 1104 reduce pain in a subject to which said extract or said one or more biologically active components have been administered.

This effect was confirmed in a *C*. *elegans* assay, wherein nematodes exposed to an extract obtained from cultivar MGC 1101 were found to be less responsive to a repetitive pain signal (see Example 4). It was further found in over 25 humans, that smoking of Cannabis flowers of cultivar MGC 1104 comprising was effective in killing pain (Example 7).

Accordingly, in a particularly preferred embodiment, the invention relates to an extract or one or more biologically active components obtainable by a method according to the invention, wherein said Cannabis flower material and/or Cannabis leaf material was obtained from Cannabis cultivar MGC 1101 and/or MGC 1104 for use in a method of treating pain in a subject in need thereof.

Said extract or one or more biologically active components may be administered to any subject in need thereof, i.e. a subject suffering from pain, in particular physical pain. Said pain may have any cause, for example, may be the result of an injury, surgery or a disease, such as flue or fever. Said pain may further be experienced in any body part, for example, in the head, e.g. headache, in a limb, e.g. caused by a cut, a bruise or the like, in the back, stomach, neck or the like. Said subject is preferably a mammal, more preferably a human suffering from pain.

It was further found that an extract or one or more biologically active components obtainable by a method according to the invention, wherein said Cannabis flower material and/or Cannabis leaf material was obtained from Cannabis cultivars MGC 1003, MGC 1106, MGC 1007, MGC 1009, MGC 1013, MGC 1027, MGC 1122, MGC 1074 and MGC 1046 relaxes or stimulates brain activity in a subject to which the extract has been administered.

This effect was confirmed in a *C*. *elegans* assay, wherein nematodes that were exposed to a repellent odor were 3-5 seconds slower in moving away from the odor when exposed to an extract obtained from cultivar MGC 1003, MGC 1007, MGC 1009, MGC 1013, MGC 1027, MGC 1122 or MGC 1074. (see Example 3). It was further empirically observed in more than 20 subjects that humans felt more relaxed, experienced fewer (disturbing) thoughts and less stress upon administration of an extract obtained from cultivar MGC 1003, MGC 1007, MGC 1009, MGC 1013 and MGC 1122. In addition, it was observed in more than 20 subjects that smoking of Cannabis flowers obtained from cultivar MGC 1104, MGC 1010, MGC 1074 and MGC 1106 lead to a stimulation of the brain, in terms of increased focus or increased concentration, increased creativity, increased feeling of alertness, increased feeling of euphoria and/or increased attention (Example 7).

Accordingly, in a particularly preferred embodiment, the invention relates to an extract or one or more biologically active components obtainable by a method according to the invention, wherein said Cannabis flower material and/or Cannabis leaf material was obtained from Cannabis cultivar MGC 1003, MGC 1104, MGC 1007, MGC 1106, MGC 1009, MGC 1010, MGC 1013, MGC 1023, MGC 1122 and/or MGC 1074 for use in a method of relaxing or stimulating brain activity in a subject in need thereof.

Such a method may be particularly advantageous in subjects suffering from disorders associated with lack of focus and/or occurrence of excessive (disturbing) thoughts. These symptoms typically occur in behavioral disorders, wherein lack of focus or the presence of negative thoughts may negatively impact mood or general wellbeing of a subject. Further, such symptoms may be a cause of sleeping disorders, e.g. wherein negative thoughts or general feeling of stress may cause problems with falling or maintaining asleep.

The invention therefore preferably relates to an extract or one or more biologically active components obtainable by a method according to the invention, wherein said Cannabis flower material and/or Cannabis leaf material was obtained from Cannabis cultivar MGC 1003, MGC 1104, MGC 1007, MGC 1009, MGC 1010, MGC 1013, MGC 1106, MGC 1023, MGC 1027, MGC 1122 and/or MGC 1074 for use in a method of treating a sleeping disorder and/or a behavioral disorder in a subject in need thereof, preferably wherein said behavioral disorder is selected from the group of major depression, bipolar disorder, anxiety disorder, obsessive compulsive disorder (OCD), attention deficit hyperactivity disorder (ADHD), attention deficit disorder (ADD), autism, schizophrenia, stress, burn-out, post-traumatic stress disorder and phobia and/or wherein said sleeping disorder is selected from night terrors and insomnia.

It was further observed in more than 10 human subjects that an extract or one or more biologically active components obtained from cultivar MGC 1003, MGC 1007, MGC 1009, MGC 1023 and MGC 1046 relaxes or stimulates the body, in particular muscles.

This effect was confirmed in a *C*. *elegans* assay, wherein motility of nematodes was increased in present of an ethanol extract obtained from cultivar MGC 1013, whereas lower motility was observed for nematodes grown in presence of an extract obtained from cultivar MGC 1101, MGC 1003, MGC 1007, MGC 1009, MGC 1027 and MGC 1046, compared to a control (see Example 5).

This effect was further confirmed in a *C*. *elegans* assay, wherein resistance to a stress was increased in nematodes that were exposed to a heat shock in presence of an ethanol extract obtained from cultivar MGC 1104 or MGC 1009 (Example 6).

It was further empirically observed in more than 20 subjects that humans felt more energetic, upon smoking of Cannabis flowers obtained from cultivar MGC 1106, whereas administration of an extract obtained from cultivar MGC 1003, MGC 1007, MGC 1009, MGC 1023 and MGC 1046 lead to relaxation of the body, in particular relaxation of the muscles (Example 7).

Accordingly, in a particularly preferred embodiment, the invention relates to an extract or one or more biologically active components obtainable by a method according to the invention, wherein said Cannabis flower material and/or Cannabis leaf material was obtained from Cannabis cultivar MGC 1101, MGC 1003, MGC 1104, MGC 1007, MGC 1009, MGC 1013, MGC 1027 and/or MGC 1046 for use in a method of relaxing or stimulating the body, preferably in stimulating or relaxing muscles in a subject in need thereof.

Such a method may be particularly advantageous in a subject, preferably a mammal, more preferably a human, suffering from a disease or disorder associated with lack of energy, such as fatigue or associated with tension of the body, such as muscle cramps, tremors, tics and muscle spasms.

Accordingly, the invention further relates to an extract or one or more biologically active components obtainable by a method according to the invention, wherein said Cannabis flower material and/or Cannabis leaf material was obtained from Cannabis cultivar MGC 1101, MGC 1003, MGC 1104, MGC 1007, MGC 1009, MGC 1013, MGC 1027 and/or MGC 1046 for use in a method of treating multiple sclerosis, Parkinson's disease, Gilles de la Tourette, amyotrophic lateral sclerosis (ALS), epilepsy and spinal cord injury.

The invention therefore preferably relates to an extract or one or more biologically active components obtainable by a method according to the invention, wherein said Cannabis flower material and/or Cannabis leaf material was obtained from Cannabis cultivar MGC 1101, MGC 1003, MGC 1104, MGC 1007, MGC 1009, MGC 1013, MGC 1027 and/or MGC 1046 for use in a method of treating muscle cramps, muscle spasm, tremors, tics or fatigue.

It was further observed that an extract or one or more biologically active components obtained from cultivar MGC 1106 increases libido in a subject.

This effect was confirmed in a *C*. *elegans* assay, wherein nematodes produced significantly more eggs in presence of an extract obtained from cultivar MGC 1106 (see Example 5). It was further empirically observed in more than 20 subjects that humans experienced an increased libido, upon administration of an extract obtained from cultivar MGC 1106, whereas this effect was absent upon smoking of cannabis flowers obtained from other cultivars (Example 7).

Increasing libido may be particularly useful in subjects suffering from a sexual arousal disorder, i.e. a disorder characterized by a lack of response to a sexual stimulation or a sexual reproduction disorder, i.e. a disorder characterized by a reduced ability to reproduce, compared to healthy controls. The invention therefore specifically relates to an extract or one or more biologically active components obtainable by a method according to the invention, wherein said Cannabis flower material and/or Cannabis leaf material was obtained from Cannabis cultivar MGC 1106 for use in treating a sexual arousal and reproduction disorder.

The invention further relates to an extract or one or more biologically active components obtainable by a method according to the invention, wherein said Cannabis flower material and/or Cannabis leaf material was obtained from Cannabis cultivar MGC 1101 for use in treating nausea and to an extract or one or more biologically active components obtainable by a method according to the invention, wherein said Cannabis flower material and/or Cannabis leaf material was obtained from Cannabis cultivar MGC 1013 for use in treating addiction.

Said addiction may be an addiction or dependency to any substance or habit, such as drug or medicine addiction (or substance use disorder), e.g. addiction or dependency to alcohol, cocaine, heroin, XTC, nicotine, amphetamines, barbiturates, benzodiazepines, pain killers, cannabinoids and the like. Addiction may also include addiction to food, gambling, gaming, sex, internet, and the like.

The invention further relates to a method of treatment by therapy of a subject in need thereof, comprising administering a therapeutically effective amount of an extract obtainable by a method according to the invention, one or more biologically active components isolated therefrom, or a pharmaceutical composition according to the invention to a subject in need thereof, preferably a human in need thereof.

Said extract, one or more biologically active components or pharmaceutically composition are as defined herein above.

Said extract, one or more biologically active components or pharmaceutical composition according to the invention may be administered via any suitable route of administration known in the art.

Examples of routes of administration include injection, oral administration, inhalation, transdermal application or rectal administration. Preferably, said extract, one or more biologically active components or pharmaceutical composition according to the invention is administered via oral administration, inhalation or transdermal application.

Further, said extract, one or more biologically active components or pharmaceutical composition according to the invention may be administered parenterally, such as intraarticularly, intravenously, intraperitoneally, subcutaneously or intramuscularly, orally, sublingually, buccally, intravitreally, nasally, topically or transdermally.

Preferably, the invention relates to a method of maintaining body weight, relaxing or stimulating energy, muscles or brain activity, inhibiting nausea, addiction or pain, reducing inflammation or reducing infection. More preferably, the invention relates to a method of treatment of nausea, addiction, sleeping disorders, eating disorders, pain, sexual arousal and reproduction disorders, fatigue, muscle cramp, muscle spasms, tics, tremors, infection, inflammation, immune system defects, neurodegenerative disorders and behavioral disorders.

The invention further relates to a use of an extract obtainable by a method according to the invention, one or more biologically active components isolated therefrom in the preparation of a medicament for treatment of a subject, preferably human in need thereof. Herein, said extract or one or more biologically active components are as defined herein above. Further, said treatment is preferably as defined herein above.

### Non-medical use

The invention further relates to a non-medical use of the one or more biologically active components or a nutritional composition according to the invention as a relaxing agent, a stimulating agent, preferably a stimulating agent to increase energy, to increase focus, to enhance creativity, to increase libido or for increasing or decreasing body weight.

Said use comprises administration of said one or more biologically active components to a healthy subject, preferably a subject that is not suffering from nausea, addiction, a sleeping disorder, an eating disorder, pain, a sexual arousal or reproduction disorder, fatigue, muscle cramps, muscle spasms, tremors, tics, an infection, inflammation, an immune system defect, a neurodegenerative disorder and a behavioral disorder. For example, one or more biologically active components according to the invention may be administered for cosmetic or nutritional purposes.

Hence, in an aspect of the invention, said one or more biologically active components or said nutritional composition is preferably administered to subjects aiming to increase or decrease body weight for aesthetic purposes, e.g. to modify the shape of the body. Preferably, said one or more biologically active components or said nutritional composition is obtained from Cannabis flowers, Cannabis leaves or parts thereof of cultivar MGC 1101 and/or cultivar MGC 1013.

In particular, one or more biologically active components or a nutritional composition according to the invention may be administered to healthy subjects not suffering from a disease or at high risk of developing a disease associated with underweight or overweight, preferably healthy humans.

Said healthy humans preferably have a BMI in the range of 18.5 to 30, preferably in the range of 19 to 28, more preferably in the range of 20 to 25. Such subjects are not suffering from a disease, or at risk of developing a disease that is associated with having an unhealthy BMI. However, such subjects may wish to decrease or increase body weight for cosmetic purposes.

Further, said one or more biologically active components or nutritional composition is preferably administered to a subject that is not suffering from an eating disorder such as anorexia nervosa, bulimia nervosa, purging disorder, avoidance/restrictive food intake disorder (ARFID), other specified feeding or eating disorders, binge eating disorder, compulsive overeating, Prader-Willi syndrome, night eating syndrome and gourmand syndrome.

Further, said one or more biologically active components or said nutritional composition is preferably administered to a subject that does not suffer from cancer and/or HIV/AIDS, more preferably, a subject that does not receive chemotherapy, radiotherapy and/or treatment for HIV/AIDS.

Further, in accordance with the invention, one or more biologically active components or nutritional composition according to the invention may be administered to subjects aiming to increase energy, to increase focus or to enhance creativity. Preferably, said one or more biologically active components or said nutritional composition is obtained from Cannabis flowers, Cannabis leaves or parts thereof of one or more of cultivars MGC 1104, MGC 1106, MGC 1009, MGC 1010, MGC 1013 and MGC 1074.

In particular, the one or more biologically active components or nutritional composition according to the invention may be administered to healthy subjects, such as students or employees wishing to increase focus or creativity, e.g. to study for an exam or increase work-related performance or athletes, wishing to boost their energy, e.g. to increase sport-related performance.

Hence, said one or more biologically active components or nutritional composition is preferably administered to a subject that is not suffering from a behavioral disorder such as major depression, bipolar disorder, anxiety disorder, OCD, ADHD, ADD, autism, schizophrenia, stress, burn-out, post-traumatic stress disorder or phobia and/or a sleeping disorder such as night terrors or insomnia.

Further, said one or more biologically active components or nutritional composition is preferably administered to a subject that is not suffering from a disease or disorder associated with lack of energy, such as fatigue or associated with tension of the body, such as muscle cramps, tremors, tics and muscle spasms. Preferably, said one or more biologically active components or nutritional composition is administered to a subject that is not suffering from multiple sclerosis, Parkinson's disease, Gilles de la Tourette, amyotrophic lateral sclerosis (ALS), epilepsy and spinal cord injury.

Further, in accordance with the invention, said one or more biologically active components or said nutritional composition may be administered to subjects aiming to increase libido. Preferably, said one or more biologically active components or said nutritional composition is obtained from Cannabis flowers, Cannabis leaves or parts thereof of cultivar MGC 1106.

In such an application, said subject, preferably a mammal, more preferably a human, is not suffering from a sexual arousal or reproduction disorder.

Further, in accordance with the invention, said one or more biologically active components may be administered as a relaxing agent to healthy subjects.

Preferably, said one or more biologically active components or said nutritional composition is obtained from Cannabis flowers, Cannabis leaves or parts thereof of one or more of cultivars MGC 1003, MGC 1007, MGC 1009, MGC 1013, MGC 1023, MGC 1027, MGC 1122, MGC 1074 and MGC 1046.

Said healthy subjects, preferably healthy humans, are not suffering from a behavioral disorder or a sleeping disorder as defined herein above.

For example, such healthy humans may administer said one or more biological compounds, when travelling, or when in a loud or noisy environment, such as an airplane or a bus. In such circumstances, it may be beneficial to take one or more biologically active components to relax and close off from the environment.

For the purpose of clarity and a concise description, features are described herein as part of the same or separate embodiments, however, it will be appreciated that the scope of the invention may include embodiments having combinations of all or some of the features described.

The invention is demonstrated by the following examples.

### Examples

### Example 1: Extraction of Cannabis flower tops and characterization of obtained extracts

### Extraction of Cannabis flowertops

For each cultivar 3 flowertops were dried and grinded to obtain an ultrafine powder having an average particle size of between about 10 and about 100 µm. Each powderized was first extracted by using a Speed Extractor E-916 (BUCHI Labortechnik AG, Flawil, Switzerland) based on the method described by Bayona et al. (Bayona et al., 2018. Mar. drugs 16:393). Briefly 1000 mg of dried and homogenized flowertop powder was mixed with 5 mL fat free quartz sand (0.3-0.9 mm, BUCHI Labortechnik AG, Flawil, Switzerland). The mixture was first extracted with 15 mL 100% *n*-hexane (VWR Chemicals, the Netherlands) in 10 mL stainless steel extraction cells at 45 °C and 100 bar. Two cycles (heat-up time of 1 min, hold time of 1 min for cycle 1 and 2 min for cycle 2, and discharge time of 2 min) were performed. The n-hexane were divided in two equal parts and the n-hexane was removed by evaporation under nitrogen. One part was heated to 143 °C for 7 minutes in a sand bath to allow decarboxylation of the acidic cannabinoids.

The remaining Cannabis flower material in the extraction cells was subsequently extracted with a mixture of 70% ethanol in water (J.T. Baker, USA and Honeywell Riedel de Haen, the Netherlands) under the same conditions as described herein above. The ethanol extracts were evaporated under reduced pressure.

### NMR analysis

Extracts were transferred to 2 mL Eppendorf tubes and dissolved in 1 ml CD₃OD. The extracts were vortexed vigorously and sonicated for 5 min. After centrifugation at 13,000 rpm for 10 min, 300 pL of the supernatant were transferred to 3 mm-NMR tubes for ¹H-NMR measurement. ¹H-NMR analysis was performed using the method described by López-Gresa et al. (López-Gresa et al., 2012. J. Plant Physiol. 169:1586- 1596). Briefly, ¹H-NMR spectra were recorded at 25 °C on a 600 MHz Bruker AV 600 spectrometer equipped with a TCI cryo-probe. CD₃OD-*d*₄ was used for the internal lock. Each ¹H-NMR spectrum consisted of 64 scans requiring 5 min acquisition time. The experimental parameters were as follows; 0.25 Hz/point, pulse width (PW) = 30° (10.8 µs), and relaxation delay (RD) = 1.5 s. Free induction decay (FIDs) were Fourier transformed with Line Broadening (LB) = 0.3 Hz and the spectra were zero-filled to 32 K points. The resulting spectra were manually phased and baseline corrected, and calibrated to CD₃OD-*d*₄ at 3.30 ppm, using Topspin (version 3.5, Bruker).

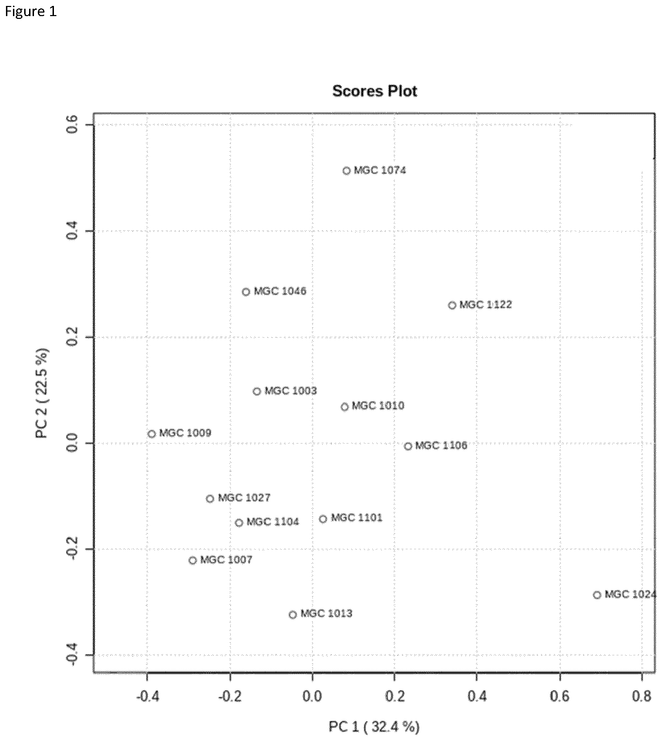

The NMR spectra of the extracts showed that the hexane extracts were enriched in cannabinoids, terpenes and sterols whereas the ethanol extracts were enriched in flavonoids. Figure 1 shows a Principale Component Analysis (PCA) of the ethanol fraction of the flower tops of the 13 different cultivars. The results of the PCA shows that the cultivars can be clearly separated from each other based on their chemical profiles.

### Example 2: Assessment of effect on eating behavior of ethanol and hexane extracts obtained from fourteen different varieties of Cannabis in Caenorhabditis elegans (C. elegans) assay

Cannabis cultivars MGC 1101, MGC 1003, MGC 1104, MGC 1106, MGC 1007, MGC 1010, MGC 1013, MGC 1023, MGC 1024, MGC 1027, MGC 1122, MGC 1074 and MGC 1046 were obtained from a medicinal grade cannabis breeding program. These cultivars were selected based on variance in genetic background and cannabinoid levels. The cultivars are commercially available from MariPharm B.V., Nieuw-Mathenesserstraat 33, Rotterdam, the Netherlands.

### Cannabis sativa influences C. elegans pharyngeal pumping in a variety specific manner

Dried ethanol and hexane extracts obtained from the flower tops of cultivars MGC 1101, MGC 1003, MGC 1104, MGC 1010, MGC 1106, MGC 1007, MGC 1009, MGC 1013, MGC 1027, MGC 1122, MGC 1074 and MGC 1046 according to the method described in Example 1 were redissolved in ethanol, and subjected to a C. *elegans* pharyngeal pumping assay as described by Raizen et al., (Raizen et al. Methods for measuring pharyngeal behaviors. In: WormBook: The Online Review of C. elegans Biology [Internet]. Pasadena (CA): WormBook; 2005-2018. Available from: https://www.ncbi.nlm.nih.gov/books/NBK126648/; accessed 5 August 2021).

To measure pharyngeal pumping after treatment with an ethanol extract, nematodes were grown from L1 stage until young adult stage on Nematode Growth Medium agar plates (3 g NaCl, 2.5 g peptone, 17 g agar, 1ml 1M MgSO₄, 1 ml 5mg/ml cholesterol, 1ml 1M CaCl₂, 25ml 1M KPO₄ buffer pH 6), adapted from Stiernagle et al. Maintenance of C. elegans (February 11, 2006), WormBook, ed. The C. elegans Research Community, WormBook) containing 100 pg/ml of the ethanol extract.

Pharyngeal pumping (neuromuscular contraction in the presence of food) was determined by measuring the number of pharyngeal pumps (contractions)/minute. Fifteen nematodes were scored for each concentration of extract for each timepoint.

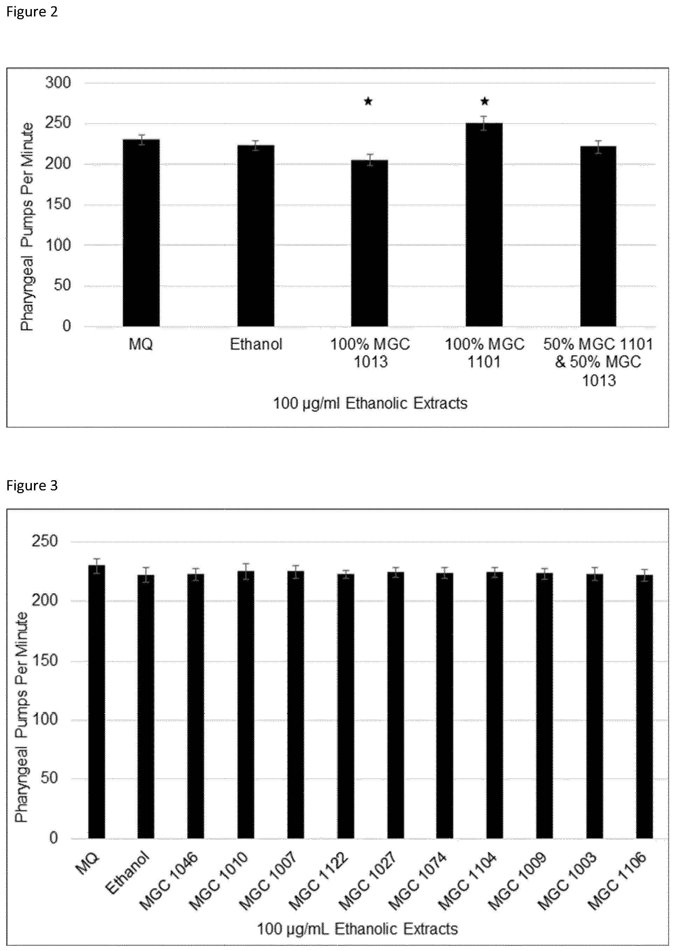

It was found that in presence of the ethanol extract obtained from cultivar MGC 1013 pharyngeal pumping of nematodes was reduced, whereas in presence of the polar extract obtained from cultivar MGC 1101, pharyngeal pumping of nematodes was increased. Notably, the effects were only observed in the polar extracts, but were absent in the hexane extracts (Figure 2).

To the contrary, no significant effect on pharyngeal pumping was observed in presence of either polar or non-polar extracts obtained from cultivars MGC 1003, MGC 1104, MGC 1010, MGC 1106, MGC 1007, MGC 1009, MGC 1027, MGC 1122, MGC 1074, MGC 1046. (Figure 3).

### Extracts from cultivars MGC 1101 and MGC 1013

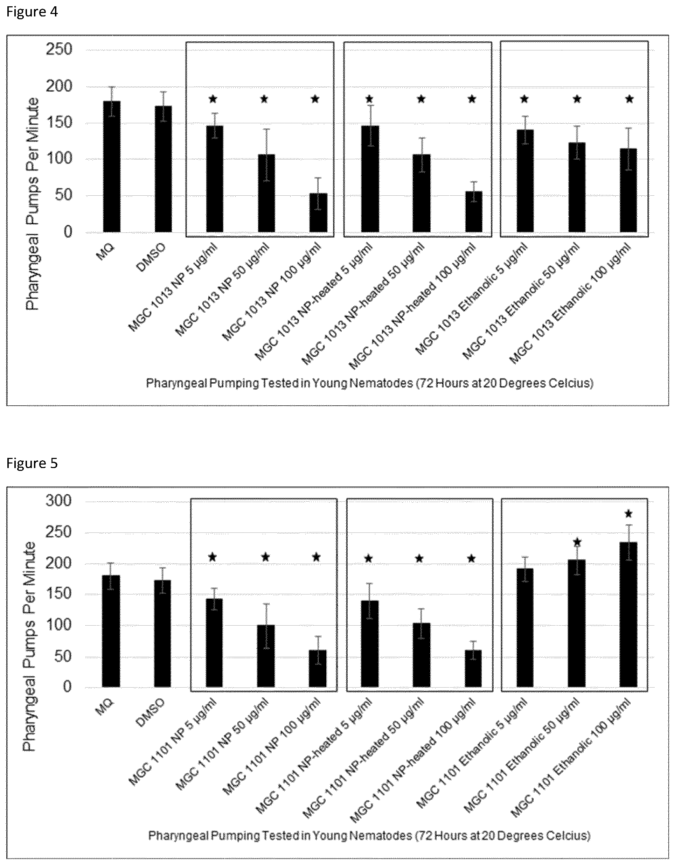

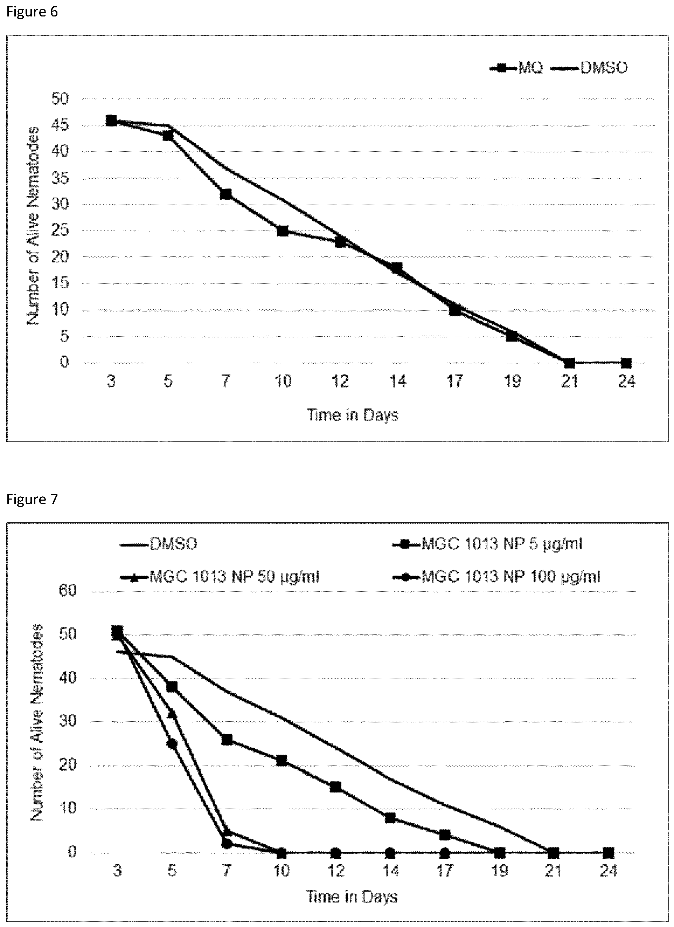

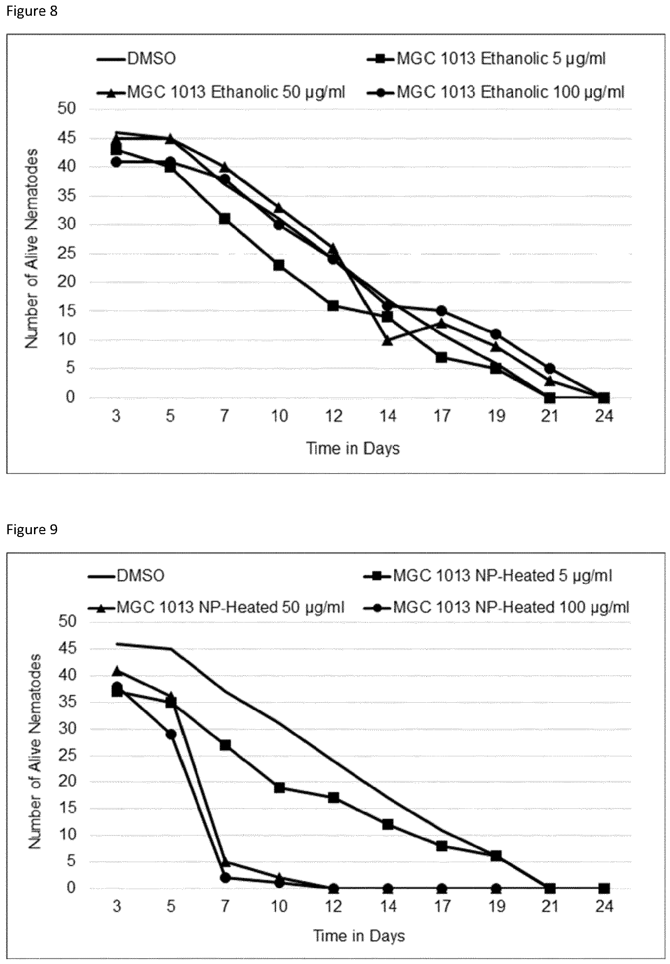

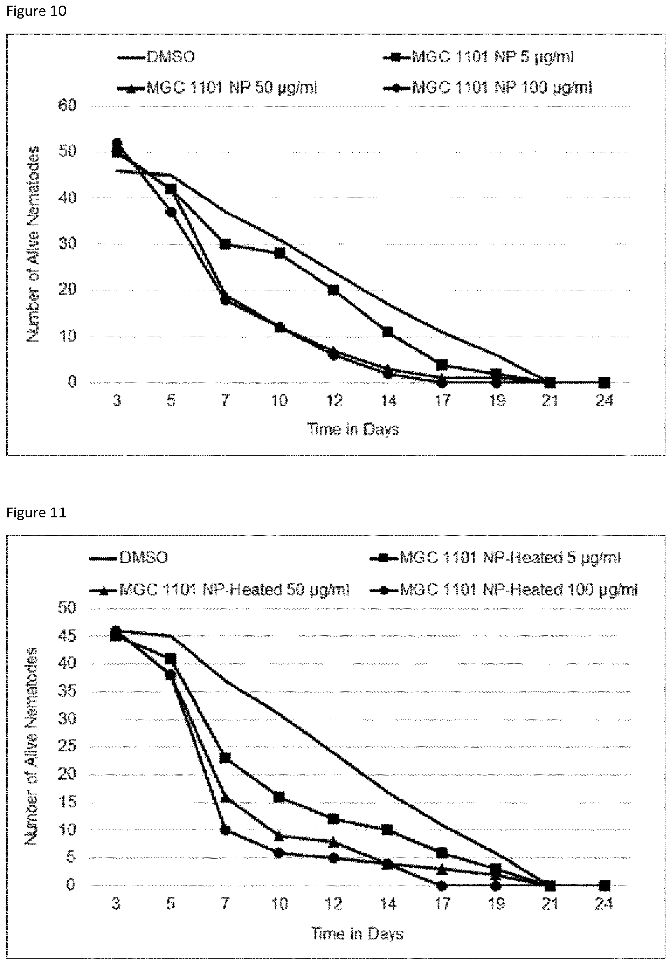

To study the effect of the ethanol extracts obtained from cultivars MGC 1101 and MGC 1013 further, nematodes were grown from L1 stage until young adult stage on solid agar plates containing 5 pg/ml, 50 pg/ml or 100 pg/ml of the heated hexane and unheated hexane extract or 5 pg/ml, 50 pg/ml or 100 pg/ml of the polar ethanol extract obtained from cultivars MGC 1101 and MGC 1013. A dose-dependent effect on pharyngeal pumping was found for both cultivars (Figure 4 and 5). Surprisingly the heated and non-heated extracts of MGC 1013 and MGC 1101 both showed a dose dependent decrease of pharyngeal pumping activity. However this was due to the fact that high doses of the non-polar extracts were toxic for the nematodes. No toxicity was observed for the ethanolic extracts up to 100 pg/ml (Figure 6-12).

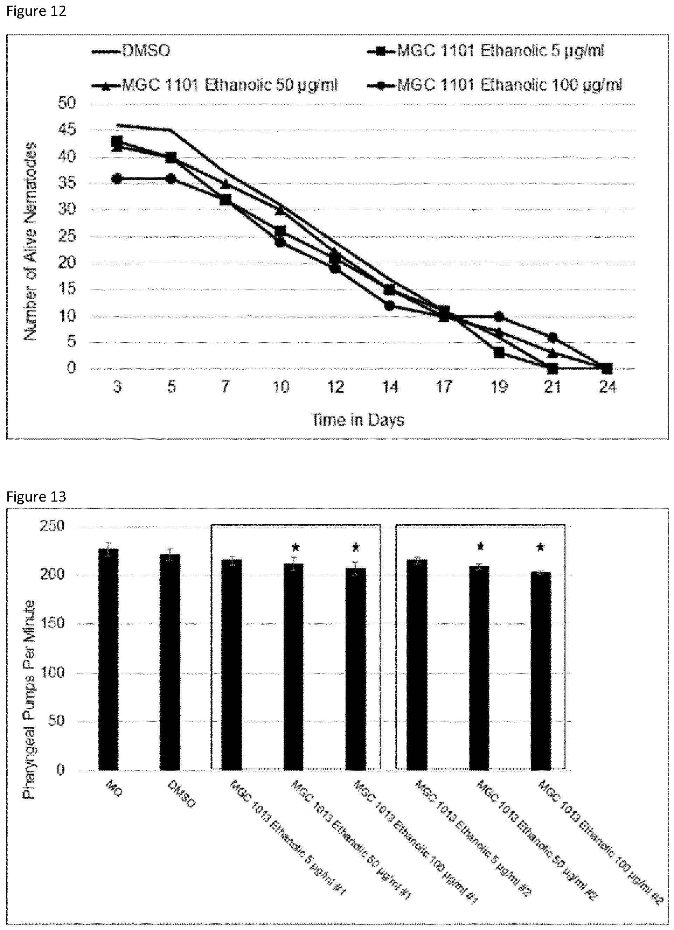

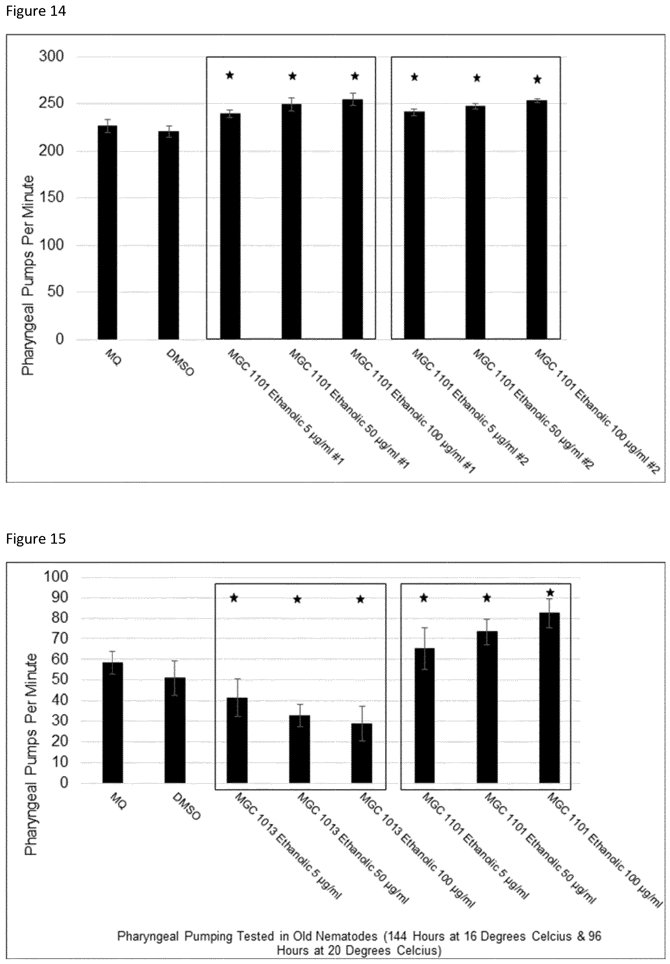

To investigate whether the pharyngeal pumping effects were reproducible, the experiments were repeated by using different flowers from both cultivars MGC 1013 and MGC 1101. Indeed the effects on pharyngeal pumping could also be observed in ethanolic extracts of different flowers (Figures 13 and 14).

Interestingly, when extracts obtained from cultivars MGC 1013 and MGC 1101 were combined to a total final concentration of 100 pg/ml, the pharyngeal pumping effects of both cultivars disappeared. This suggests that both cultivars might affect a similar molecular mechanism yet in an opposite way (Figure 2).

Pharyngeal pumping rate is known to decrease when nematodes become older. To investigate if the cultivars were able to robustly influence pharyngeal pumping also in an aging nematode population, nematodes were again grown on agar plates with the cannabis extracts from L1 stage onwards. Pharyngeal pumping was tested when nematodes were 12 days old.

The pharyngeal pumping rate was indeed reduced in aging nematodes compared to young ones (compare Figure 4 & 5 with Figure 15). As can be derived from Figure 15, the pharyngeal pumping rate was enhanced in presence of an ethanol extract obtained from cultivar MGC 1101, while pharyngeal pumping rate was reduced in presence of an ethanol extract obtained from cultivar MGC 1013. Thus, even in aging nematode populations ethanol extracts obtained from cultivars MGC 1101 and MGC 1013 are able to influence pharyngeal pumping.

Pharyngeal pumping of *C*. *elegans* constitutes a good model for eating behaviour, because pharyngeal pumping occurs during food intake of the *C. elegans* and is influenced by previous food experiences, such as starvation and/or food quality. (Avery et al. 2012 C. elegans feeding. The C. elegans Research Community. Wormbook 1551-8507 101895/wormbook 11501, Trojanowski et al. 2016. Sci Rep 6:22940).

Thus, from these results it can be concluded that an ethanol extract obtained from flower tops of cultivar MGC 1101 increase food consumption, whereas an ethanol extract obtained from flower tops of cultivar MGC 1013 reduce food consumption of C. *elegans.* Surprisingly, these effects are independent of the presence of cannabinoids, terpenes and/or steroids, since no such effects were obtained for the hexane extract - typically comprising these components - of flower tops.

### Example 3: Assessment of effect on brain activity of ethanol extracts obtained from varieties MGC 1101, MGC 1003, MGC 1106, MGC 1007, MGC 1009, MGC 1013, MGC 1027, MGC 1122, MGC 1074 and MGC 1046 of Cannabis in a C. elegans assay

The nervous system is responsible for the integration of external stimuli in order to induce appropriate behavioural and mental responses. Like other living organisms, *C. elegans* has to integrate external stimuli in order to respond in an appropriate manner to its surroundings. Mental relaxation can therefore be defined by the reduced response towards negative environmental signals that depend on their integration through the nervous system. This effect was assesses by monitoring the response of nematodes towards a noxious smell.

### Response of nematodes towards smell of 1-Octanol

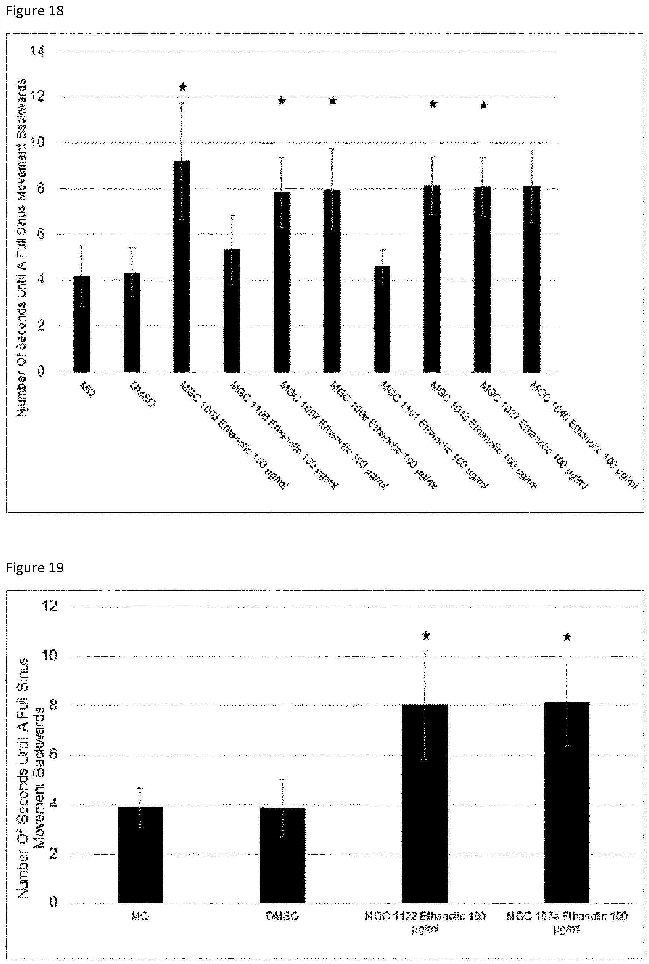

*1-Octanol* reversal tests were performed according to the method reported by Chao et al. and Harris et al. (Chao et al., 2004. Proc Natl Acad Sci USA 101:15512-15517; Harris et al., 2011. PLoS One 6:e21897). 1-octanol is a repellent odor for *C. elegans* and nematodes respond to the presence of 1-octanol by moving away from it when placed at one body distance in front of the nose of the nematode. Nematodes were grown at 20 °C on agar plates in presence of ethanol extract. Cannabis extracts obtained from flower tops of cultivars MGC 1003, MGC 1101, MGC 1106, MGC 1007, MGC 1009, MGC 1013, MGC 1027, MGC 1046, MGC 1122 and MGC 1074. Young adult nematodes were placed on agar in 6 cm dishes to execute the assay. An eyelash pick was dipped in 30% 1-Octanol and positioned at body length distance in front of a moving nematode. Time until nematodes completed a sinusoidal backward movement was monitored. The experiment was performed in duplicate, using 15 nematodes per experiment (total 30 nematodes). The results are shown in table 1 and Figures 18 and 19.

**Table 1; effects of extracts obtained from cultivars on response of nematodes to smell of 1-Octanol nematodes;**

| Cultivar | Concentration (µ/mL) | Extract | Average time to reversal after 1-Octanol (s) | Significance compared to control |
|---|---|---|---|---|
| Control | 0 | | 4.2 ± 0.4 | - |
| MGC 1101 | 100 | Ethanol | 4.6 | No |
| MGC 1003 | 100 | Ethanol | 9.2 | Yes |
| MGC 1106 | 100 | Ethanol | 5.3 | No |
| MGC 1007 | 100 | Ethanol | 7.8 | Yes |
| MGC 1009 | 100 | Ethanol | 8.0 | Yes |
| MGC 1013 | 100 | Ethanol | 8.1 | Yes |
| MGC 1027 | 100 | Ethanol | 8.1 | Yes |
| MGC 1122 | 100 | Ethanol | 8.0 | Yes |
| MGC 1074 | 100 | Ethanol | 7.8 | Yes |
| MGC 1046 | 100 | Ethanol | 6.2 | No |

It was found that the ethanol extracts obtained from flower tops of cultivars MGC 1003, MGC 1007, MGC 1009, MGC 1013, MGC 1027, MGC 1122 and MGC 1074 had an effect on reducing the response of nematodes towards 1-Octanol (Table 1 and Figures 18 and 19).

This data suggest that nematodes are more relaxed in presence of an extract obtained from flower tops of cultivars MGC 1003, MGC 1007, MGC 1009, MGC 1013, MGC 1027, MGC 1122 and MGC 1074 because they are more tolerant towards undesired circumstances such as the presence of an unpleasant smell.

### Example 4: Assessment of inhibition of pain of ethanol extracts obtained from varieties MGC 1101 and MGC 1013 of Cannabis in a C. elegans assay

### Noxious stimulus (nose tap)

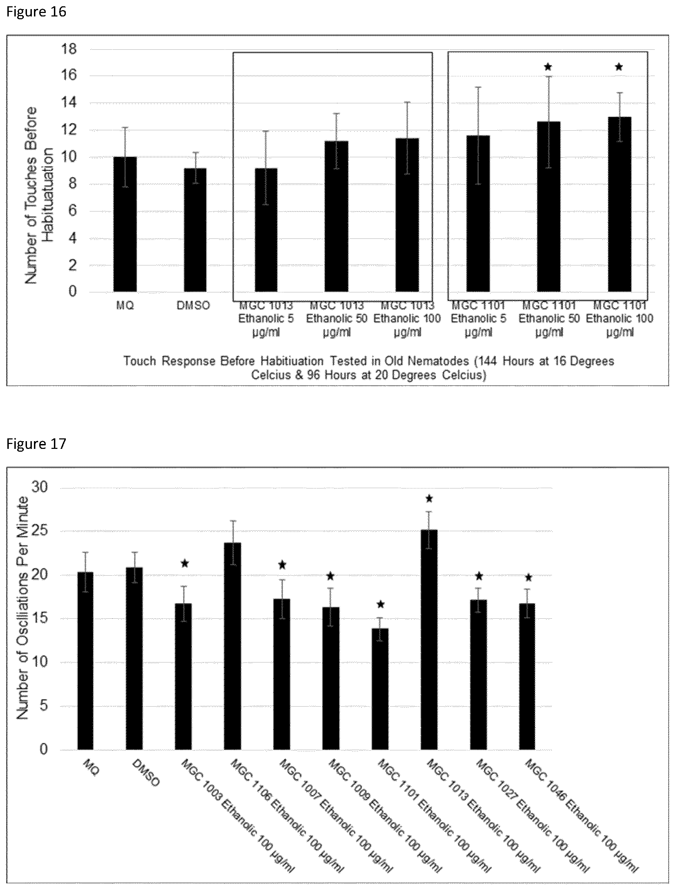

Repetitive nose taps were applied according to method described by Chalfie et al., (Chalfie M, Hart AC, Rankin CH, et al. Assaying mechanosensation. In: WormBook: The Online Review of C. elegans Biology [Internet]. Pasadena (CA): WormBook; 2005-2018. Available from: https://www.ncbi.nlm.nih.gov/books/NBK235860/; accessed on 3 August 2021). Nematodes were grown on agar plated that contained a polar extract obtained from cultivar MGC 1101 or MGC 1013 during L1 to young adult stage. The adaptive response to a noxious stimulus (non-associated learning) was measured by giving nematodes a repetitive touch/noxious stimulus and by scoring their response. Ten nematodes were scored for each condition for each timepoint/treatment group/sample. The results are shown in Figure 16.

It was found that nematodes grown in presence of a polar extract obtained from cannabis flower and/or leaf material obtained from cultivar MGC 1101 were less responsive to a repetitive pain signal. No significant effect was obtained for nematodes grown in presence of a polar extract obtained from cannabis flower and/or leaf material obtained from cultivar MGC 1013.

### Example 5: Assessment of motility and reproduction capacity of ethanol extracts obtained from cultivar MGC 1101, MGC 1003, MGC 1104, MGC 1106, MGC 1007, MGC 1009, MGC 1010, MGC 1013, MGC 1027, MGC 1122, MGC 1074 and MGC 1046 of Cannabis in a C. elegans assay

To determine motility, the total number of complete body bends (oscillations) per minute were counted. Nematodes were grown on agar plates that contained ethanol extracts obtained from cannabis flower and/or Cannabis leaf material obtained from cultivar MGC 1003, MGC 1104, MGC 1106, MGC 1007, MGC 1009, MGC 1010, MGC 1013, MGC 1027, MGC 1122, MGC 1074 and MGC 1046 during L1 to young adult stage. To investigate motility the number of complete body bends (oscillations) per minute were scored on new plates next to the bacterial food in which they were allowed to acclimate for 30 seconds before oscillations were scored. Ten nematodes were scored for each condition for each timepoint/ treatment group. The results are shown in Table 2 and Figure 17.

**Table 2; effects of extracts obtained from cultivars on motility and reproduction capacity of nematodes; -- much less than average, - less than average, +/- average, + more than average; ++ much more than average**

| Cultivar | Concentration (µ/mL) | Extract | Average number of oscillations/minute | Significance compared to control | Number of eggs |
|---|---|---|---|---|---|
| Control | 0 | | 23.9 ± 4.4 | - | |
| MGC 1101 | 100 | Ethanol | 13.7 | yes | +/- |
| MGC 1003 | 100 | Ethanol | 16.7 | yes | +/- |
| MGC 1104 | 100 | Ethanol | +/- | | +/- |
| MGC 1106 | 100 | Ethanol | 23.6 | No | ++ |
| MGC 1007 | 100 | Ethanol | 17.2 | yes | +/- |
| MGC 1009 | 100 | Ethanol | 16.2 | yes | +/- |
| MGC 1010 | 100 | Ethanol | +/- | | +/- |
| MGC 1013 | 100 | Ethanol | 25.1 | yes | |
| MGC 1027 | 100 | Ethanol | 17.1 | yes | |
| MGC 1122 | 100 | Ethanol | 27.8 | No | +/- |
| MGC 1074 | 100 | Ethanol | 28.4 | No | +/- |
| MGC 1046 | 100 | Ethanol | 16.7 | yes | +/- |

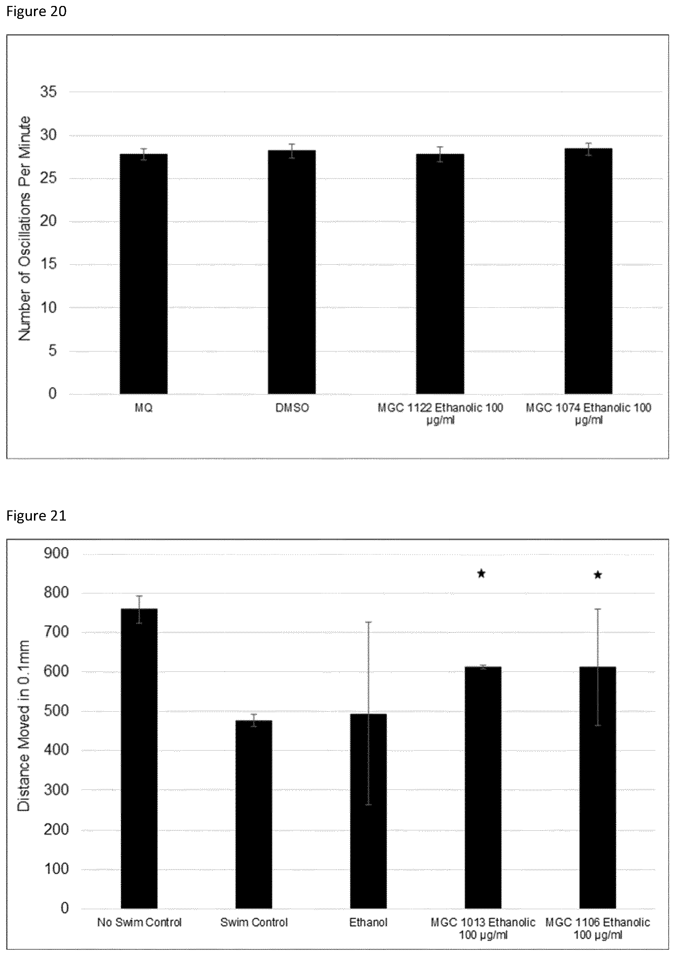

It can be derived from table 2, that ethanol extracts obtained from Cannabis flower and/or leaf material of cultivar MGC 1013 has a stimulating effect on the body, as reflected by a significantly higher motility of nematodes compared to the control. This was also confirmed in an endurance assay wherein nematodes were exhausted during a swimming period (swim control) in the presence of 100 µg/mL ethanolic extract of cultivars MGC 1013 and MGC 1106. As shown in Figure 21, exposure to both cultivars 1013 and 1106 resulted in a higher endurance as compared to the control solvent (ethanol).

It can also be derived from table 2 that ethanol extracts from MGC 1101, MGC 1003, MGC 1007, MGC 1009, MGC 1027 and 1046 have a relaxing effect on the body, as reflected in a significantly lower motility of C. *elegans* compared to the control (See Figure 17).

A polar extract obtained from cultivar MGC 1106 was found to have a stimulating effect on the libido or fertility of C. *elegans,* as indicated by a much higher production of eggs in presence of an extract obtained from this cultivar (Table 2).

### Example 6: Assessment of resistance to heat stress of nematodes in presence of ethanol extracts obtained from varieties MGC 1101, MGC 1104 and MGC 1009 in a C. elegans assay

### Survival rate after heat stress

Life span after heat stress was measured by adding an ethanol extract obtained from cannabis flower and/or Cannabis leaf material obtained from cultivar MGC 1101, MGC 1104, MGC 1009, MGC 1010, MGC 1013 and MGC 1024 into the solid medium on which the nematodes are grown. Nematodes were transferred to freshly prepared plates every two or three days to ensure the continuous presence of ethanol extracts.

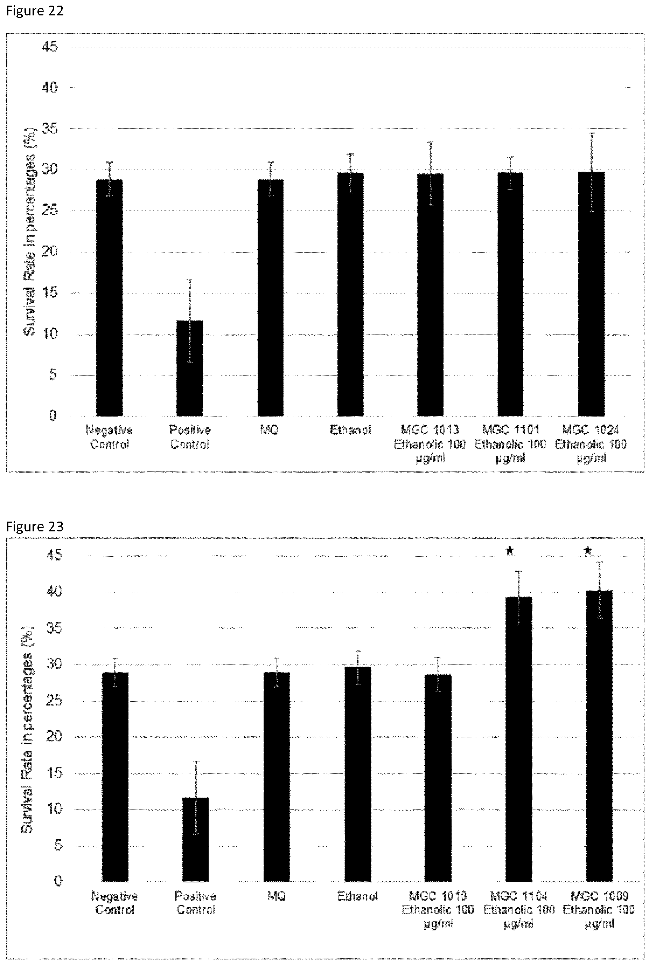

A short heat stress shock was provided to nematodes: 5 hours at 37 degrees as young adult animals. Subsequently, the nematodes were allowed to recover overnight at temperature of 20 °C. The next day the survival rate was determined. Nematodes in presence of an ethanol extract obtained from cannabis flower and/or cannabis leaf material obtained from cultivars MGC 1104 and MGC 1009 were found to be significantly more resistant against heat stress as indicated by a significantly higher survival rate (Figure 22 and 23).

From these results, it may be obtained that polar extracts obtained from Cannabis flower and/or Cannabis leaf material obtained from cultivars MGC 1104 and MGC 1009 are capable of resisting stress, because a higher survival rate was obtained for nematodes that were exposed to a heat shock.

### Example 7: In vivo assessment of biological activities of extracts obtained from cultivars in human

Human volunteers received Cannabis flowers obtained from a cultivar described in table 2. They were asked to monitor their response to smoking of the Cannabis flowers in terms of appetite, energy, pain, thoughts, libido and muscle function. The results are shown in table 3.

**Table 3; Overview of cannabis cultivars and their observed effects in human**

| Cultivars | Cannabinoid | Observed effect |
|---|---|---|
| MGC 1101 | THC-rich | Stimulating appetite |
| | | Inhibition of nausea |
| MGC 1003 | THC-rich | Relaxation of mind |
| | | Relaxation of body |
| MGC 1104 | THC-rich | Killing pain |
| | | Providing euphoria |
| MGC 1106 | THC-rich | Boosting energy |
| | | Stimulating creativity |
| | | Stimulating libido |
| MGC 1007 | THC-rich | Relaxation mind |
| | | Relaxation body |
| | | Inducing sleeping |
| MGC 1009 | THC-rich | Relaxation of mind |
| | | Relaxation of body |
| | | Providing euphoria |
| | | Inducing sleeping |
| MGC 1010 | THC-rich | Providing euphoria |
| MGC 1013 | CBD-rich | Anti-addiction |
| | | Enhanced ability to concentrate |
| | | Relaxation mind |
| | | Inhibition appetite |
| MGC 1023 | THC-rich | Relaxation mind |
| | | Relaxation body |
| | | Inducing sleeping |
| MGC 1122 | CBD-rich | Relaxation mind |
| MGC 1074 | CBD-rich | Enhanced ability to concentrate |
| MGC 1046 | THC-rich | Relaxation body |
| | | Relaxation muscles |

As can be derived from table 3, extracts obtained from cultivars MGC 1101, MGC 1003, MGC 1104, MGC 1106, MGC 1007, MGC 1009, MGC 1010, MGC 1013, MGC 1023, MGC 1122, MGC 1074 and MGC 1046 had a biological effect on humans. These effects strongly correlated to the effects observed in a *C*. *elegans* assay as described in Examples 1-6.

## Claims

1. A method for obtaining one or more biologically active components other than a cannabinoid and/or a terpene from a cannabinoid-poor and/or terpene-poor waste product comprising Cannabis flower and/or Cannabis leaf material, comprising contacting the cannabinoid-poor and/or terpene-poor waste product comprising Cannabis flower and/or Cannabis leaf material with a polar solvent, thereby obtaining an extract comprising one or more biologically active components and optionally isolating said one or more biologically active components from said extract.

2. The method according to claim 1, wherein said Cannabis flower and/or leaf material comprises Cannabis flowers, Cannabis leaves or parts thereof obtained from the species *Cannabis sativa* L.

3. The method according to claim 1 or 2, wherein said Cannabis flowers, Cannabis leaves or parts thereof are obtained from a Cannabis variety chosen from the group selected from MGC 1101, MGC 1002, MGC 1003, MGC 1104, MGC 1005, MGC 1106, MGC 1007, MGC 1009, MGC 1010, MGC 1013, MGC 1023, MGC 1024, MGC 1027, MGC 1122, MGC 1074 and MGC 1046.

4. The method according to any one of the preceding claims, wherein said Cannabis flower and/or leaf material comprises Cannabis flower buds or parts thereof, preferably dried Cannabis flower buds or parts thereof.

5. The method according to any one of the preceding claims, wherein said one or more biologically active components comprises at least one component having a stimulating or a relaxing effect, preferably a stimulating effect in enhancing appetite, energy, muscle function and/or brain activity, and/or a relaxing effect, preferably relaxing muscle tension or brain activity, inhibition of nausea, inhibition of pain or inhibition of addiction.

6. The method according to any one of the preceding claims, wherein said one or more biologically active components is polar and/or wherein said one or more biologically active component is water-soluble, preferably has a water solubility of at least 1 mg per 100 mL.

7. The method according to any one of the preceding claims, wherein the polar solvent is selected from the group consisting of water, aqueous buffer, methanol, ethanol, propanol, 2-propanol, butanol, 2-methyl-propanol, acetone, ethyl acetate, dimethyl sulfoxide, dimethylformamide, acetonitrile, 1,4-dioxane, tetrahydrofuran, or a combination thereof.

8. The method according to any one of the preceding claims, wherein said cannabinoid-poor and/or terpene-poor waste product comprising Cannabis flower and/or leaf material is obtained by contacting Cannabis flowers, leaves or parts thereof with an organic non-polar solvent, thereby obtaining a cannabinoid-rich and/or terpene-rich fluid and a cannabinoid-poor and/or terpene-poor waste product comprising Cannabis flower and/or leaf material, wherein said cannabinoid-poor and/or terpene-poor waste product preferably comprises less than 10 wt.% of cannabinoids and/or terpenes, based on the dry weight of said waste product, more preferably wherein said waste product is essentially free of cannabinoids and/or terpenes, as determinable with ¹H NMR spectroscopy, preferably wherein said organic non-polar solvent is selected from the group consisting of n-pentane, n-hexane, n-heptane, n-octane, n-nonane, n-decane, n-dodecane, 2-methylpentane, 3-methylpentane, 2-methylhexane, 3-methylhexane, cyclopentane, cyclohexane, cycloheptane, cyclooctane, cyclodecane, cyclododecane, benzene, toluene, naphthalene, carbon tetrachloride, chloroform, dichloromethane, diethylether, supercritical carbon dioxide or a combination thereof.

9. The method according to any one of the preceding claim wherein said one or more biologically active components are isolated by evaporation of said polar solvent from said extract.

10. The method according to any one of the preceding claims, wherein said extract comprising one or more biologically active components further comprises one or more cannabinoids and/or terpenes, wherein said cannabinoids and/or terpenes are preferably present in an amount of at most 10 wt.% of terpenes and/or at most 10 wt.% of cannabinoids, based on the total weight of said one or more biologically active components.

11. An extract comprising one or more biologically active components or one or more biologically active components obtainable by a method according to any one of claims 1-10.

12. A pharmaceutical composition comprising one or more biologically active components according to claim 11 and a pharmaceutically acceptable carrier.

13. One or more biologically active components according to claim 11 or a pharmaceutical composition according to claim 12 for use in a method of treatment by therapy.

14. One or more biologically active components according to claim 11 or a pharmaceutical composition according to claim 12 for use in the prevention or treatment of nausea, addiction, sleeping disorders, eating disorders, pain, sexual arousal and reproduction disorders, fatigue, muscle cramps, muscle spasms, tremors, tics, infections, inflammation, immune system defects, neurodegenerative disorders and behavioral disorders.

15. The one or more biologically active components or the pharmaceutical composition for use according to claim 14, wherein said behavioral disorder is selected from the group consisting of major depression, bipolar disorder, anxiety disorder, attention deficit hyperactivity disorder (ADHD), attention deficit disorder (ADD), autism, schizophrenia, stress, burn-out, post-traumatic stress disorder and phobia.

16. Use of the one or more biologically active components according to claim 10 as a relaxing agent, to increase energy, to increase focus, to enhance creativity, to enhance libido, or for increasing or decreasing body weight.
